# EUROPEAN PATENT APPLICATION

(11) **EP 4 079 841 A1**
(43) Date of publication of application: **26.10.2022**
(21) Application number: 20902832.3
(22) Date of filing: 18.12.2020
(51) Int. Cl.: C12N 5/0783, C12N 5/10, A61K 35/17, C07K 16/28, C12N 15/13, A61P 35/00

(54) **ENGINEERED T CELL, PREPARATION THEREFOR AND USE THEREOF**

(30) Priority: 19.12.2019 CN 201911319383
(71) Applicant: Fundamenta Therapeutics Inc., Suzhou, Jiangsu 215200 (CN)
(72) Inventor: LI, Jun, Suzhou, Jiangsu 215200 (CN); ZHANG, Pengchao, Suzhou, Jiangsu 215200 (CN)
(74) Representative: EP&C
(86) International application number: PCT/CN2020/137591
(87) International publication number: WO 2021/121383

(57) **Abstract**

Provided is an engineered T cell. A polypeptide down-regulating the expression of a TCR/CD3 complex on cell surface is introduced to reduce the expression of the TCR/CD3 complex on the cell surface. The engineered T cell can be used for therapeutic purposes, such as cancer treatmenr.

SEQUENCE LISTING

<110>  FUNDAMENTA THERAPEUTICS INC.

<120>  Engineered T Cells, Preparation Thereof and Use Thereof

<130>  196265

<150>  CN 201911319383.8
<151>  2019-12-19

<160>  51

<170>  PatentIn version 3.5

<210>  1
<211>  120
<212>  PRT
<213>  Artificial Sequence

<220>
<223>  VH

<400>  1

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Tyr
            20                  25                  30

Thr Met His Trp Val Arg Gln Ala Pro Gly Gln Gly Leu Glu Trp Met
        35                  40                  45

Gly Tyr Ser Thr Pro Arg Ser Gly Tyr Thr His Tyr Asn Gln Lys Leu
    50                  55                  60

Lys Asp Lys Ala Thr Leu Thr Ala Asp Lys Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Ala Tyr Tyr Asp Tyr Asp Gly Phe Ala Tyr Trp Gly Gln
            100                 105                 110

Gly Thr Leu Val Thr Val Ser Ser
        115                 120

<210>  2
<211>  106
<212>  PRT
<213>  Artificial Sequence

<220>

<223>  VL

<400>  2

Asp Ile Gln Met Thr Gln Ser Pro Ser Ser Leu Ser Ala Ser Val Gly
1               5                   10                  15

Asp Arg Val Thr Ile Thr Cys Ser Ala Ser Ser Ser Val Ser Tyr Met
            20                  25                  30

Asn Trp Tyr Gln Gln Lys Pro Gly Lys Ala Pro Lys Arg Leu Ile Tyr
        35                  40                  45

Asp Thr Ser Lys Leu Ala Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Trp Ser Thr Gln Pro Pro Thr
                85                  90                  95

Phe Gly Gly Gly Thr Lys Val Glu Ile Lys
            100                 105


<210>  3
<211>  8
<212>  PRT
<213>  Artificial Sequence

<220>
<223>  HCDR1

<400>  3

Gly Tyr Thr Phe Ile Ser Tyr Thr
1               5


<210>  4
<211>  8
<212>  PRT
<213>  Artificial Sequence

<220>
<223>  HCDR2

<400>  4

Ser Thr Pro Arg Ser Gly Tyr Thr
1               5


<210>  5
<211>  13
<212>  PRT

<213>  Artificial Sequence

<220>
<223>  HCDR3

<400>  5

Ala Arg Ser Ala Tyr Tyr Asp Tyr Asp Gly Phe Ala Tyr
1               5                   10


<210>  6
<211>  5
<212>  PRT
<213>  Artificial Sequence

<220>
<223>  LCDR1

<400>  6

Ser Ser Val Ser Tyr
1               5


<210>  7
<211>  3
<212>  PRT
<213>  Artificial Sequence

<220>
<223>  LCDR2

<400>  7

Asp Thr Ser
1


<210>  8
<211>  9
<212>  PRT
<213>  Artificial Sequence

<220>
<223>  LCDR3

<400>  8

Gln Gln Trp Ser Thr Gln Pro Pro Thr
1               5


<210>  9
<211>  8
<212>  PRT
<213>  Artificial Sequence

<220>
<223>  HCDR2


<220>

<221>  misc_feature
<222>  (1)..(1)
<223>  The 'Xaa' at location 1 stands for any amino acid.

<220>
<221>  misc_feature
<222>  (2)..(2)
<223>  The 'Xaa' at location 2 stands for any amino acid.

<220>
<221>  misc_feature
<222>  (3)..(3)
<223>  The 'Xaa' at location 3 stands for any amino acid.

<220>
<221>  misc_feature
<222>  (4)..(4)
<223>  The 'Xaa' at location 4 stands for any amino acid.

<400>  9

Xaa Xaa Xaa Xaa Ser Gly Tyr Thr
1               5


<210>  10
<211>  8
<212>  PRT
<213>  Artificial Sequence

<220>
<223>  HCDR2

<400>  10

Ile Thr Pro Arg Ser Gly Tyr Thr
1               5


<210>  11
<211>  8
<212>  PRT
<213>  Artificial Sequence

<220>
<223>  HCDR2

<400>  11

Ile Thr Thr Leu Ser Gly Tyr Thr
1               5


<210>  12
<211>  8
<212>  PRT
<213>  Artificial Sequence

<220>
<223>  HCDR2

<400>  12

Ser Asn Pro Arg Ser Gly Tyr Thr
1               5

<210>  13
<211>  13
<212>  PRT
<213>  Artificial Sequence

<220>
<223>  HCDR3

<220>
<221>  misc_feature
<222>  (4)..(4)
<223>  The 'Xaa' at location 4 stands for any amino acid.

<220>
<221>  misc_feature
<222>  (5)..(5)
<223>  The 'Xaa' at location 5 stands for any amino acid.

<220>
<221>  misc_feature
<222>  (6)..(6)
<223>  The 'Xaa' at location 6 stands for any amino acid.

<400>  13

Ala Arg Ser Xaa Xaa Xaa Asp Tyr Asp Gly Phe Ala Tyr
1               5                   10

<210>  14
<211>  13
<212>  PRT
<213>  Artificial Sequence

<220>
<223>  HCDR3

<400>  14

Ala Arg Ser Arg Glu Ser Asp Tyr Asp Gly Phe Ala Tyr
1               5                   10

<210>  15
<211>  9
<212>  PRT
<213>  Artificial Sequence

<220>
<223>  LCDR3

<220>
<221>  misc_feature
<222>  (4)..(4)
<223>  The 'Xaa' at location 4 stands for any amino acid.

<220>

<221>  misc_feature
<222>  (5)..(5)
<223>  The 'Xaa' at location 5 stands for any amino acid.

<220>
<221>  misc_feature
<222>  (6)..(6)
<223>  The 'Xaa' at location 6 stands for any amino acid.

<220>
<221>  misc_feature
<222>  (7)..(7)
<223>  The 'Xaa' at location 7 stands for any amino acid.

<220>
<221>  misc_feature
<222>  (8)..(8)
<223>  The 'Xaa' at location 8 stands for any amino acid.

<400>  15

Gln Gln Trp Xaa Xaa Xaa Xaa Xaa Thr
1               5


<210>  16
<211>  9
<212>  PRT
<213>  Artificial Sequence

<220>
<223>  LCDR3

<400>  16

Gln Gln Trp Ser Ser Thr Val Pro Thr
1               5


<210>  17
<211>  9
<212>  PRT
<213>  Artificial Sequence

<220>
<223>  LCDR3

<400>  17

Gln Gln Trp Ala His Asn Pro Pro Thr
1               5


<210>  18
<211>  9
<212>  PRT
<213>  Artificial Sequence

<220>
<223>  LCDR3

<400>  18

Gln Gln Trp Ser Tyr Val Arg Pro Thr
1               5

<210>  19
<211>  9
<212>  PRT
<213>  Artificial Sequence

<220>
<223>  LCDR3

<400>  19

Gln Gln Trp Ser Phe Val Ser Pro Thr
1               5

<210>  20
<211>  9
<212>  PRT
<213>  Artificial Sequence

<220>
<223>  LCDR3

<400>  20

Gln Gln Trp Ala Met Ser Pro Pro Thr
1               5

<210>  21
<211>  9
<212>  PRT
<213>  Artificial Sequence

<220>
<223>  LCDR3

<400>  21

Gln Gln Trp Ser Ser Trp Pro Pro Thr
1               5

<210>  22
<211>  9
<212>  PRT
<213>  Artificial Sequence

<220>
<223>  LCDR3

<400>  22

Gln Gln Trp Ser Gln Pro His Thr Thr
1               5

<210>  23
<211>  9
<212>  PRT

<213>  Artificial Sequence

<220>
<223>  LCDR3

<400>  23

Gln Gln Trp Ser Asn Gln Pro Pro Thr
1               5


<210>  24
<211>  9
<212>  PRT
<213>  Artificial Sequence

<220>
<223>  LCDR3

<400>  24

Gln Gln Trp Leu Leu Gly Pro Pro Thr
1               5


<210>  25
<211>  9
<212>  PRT
<213>  Artificial Sequence

<220>
<223>  LCDR3

<400>  25

Gln Gln Trp Ser Thr Gln Arg Pro Thr
1               5


<210>  26
<211>  9
<212>  PRT
<213>  Artificial Sequence

<220>
<223>  LCDR3

<400>  26

Gln Gln Trp Ser Glu Arg Pro Pro Thr
1               5


<210>  27
<211>  9
<212>  PRT
<213>  Artificial Sequence

<220>
<223>  LCDR3

<400>  27

Gln Gln Trp Ser Gly Gln Pro Pro Thr
1               5

<210>  28
<211>  9
<212>  PRT
<213>  Artificial Sequence

<220>
<223>  LCDR3

<400>  28

Gln Gln Trp Lys Cys Asn Pro Pro Thr
1               5

<210>  29
<211>  9
<212>  PRT
<213>  Artificial Sequence

<220>
<223>  LCDR3

<400>  29

Gln Gln Trp Arg Asn Ala Pro Pro Thr
1               5

<210>  30
<211>  9
<212>  PRT
<213>  Artificial Sequence

<220>
<223>  LCDR3

<400>  30

Gln Gln Trp Ser Ile Gly Leu Pro Thr
1               5

<210>  31
<211>  9
<212>  PRT
<213>  Artificial Sequence

<220>
<223>  LCDR3

<400>  31

Gln Gln Trp Lys Thr Ser Pro Pro Thr
1               5

<210>  32
<211>  9
<212>  PRT

<213>  Artificial Sequence

<220>
<223>  LCDR3

<400>  32

Gln Gln Trp Ser Tyr Thr Ser Pro Thr
1               5


<210>  33
<211>  9
<212>  PRT
<213>  Artificial Sequence

<220>
<223>  LCDR3

<400>  33

Gln Gln Trp His Gly Lys Pro Pro Thr
1               5


<210>  34
<211>  9
<212>  PRT
<213>  Artificial Sequence

<220>
<223>  LCDR3

<400>  34

Gln Gln Trp Ser Thr Ser Glu Pro Thr
1               5


<210>  35
<211>  6
<212>  PRT
<213>  Artificial Sequence

<220>
<223>  Retention sequence 1

<400>  35

Ala Glu Lys Asp Glu Leu
1               5


<210>  36
<211>  4
<212>  PRT
<213>  Artificial Sequence

<220>
<223>  Retention sequence 2

<400>  36

Lys Asp Glu Leu
1


<210> 37
<211> 4
<212> PRT
<213> Artificial Sequence

<220>
<223> Retention sequence 3


<220>
<221> misc_feature
<222> (3)..(3)
<223> The 'Xaa' at location 3 stands for any amino acid.

<220>
<221> misc_feature
<222> (4)..(4)
<223> The 'Xaa' at location 4 stands for any amino acid.

<400> 37

Lys Lys Xaa Xaa
1


<210> 38
<211> 3
<212> PRT
<213> Artificial Sequence

<220>
<223> Retention sequence 4


<220>
<221> misc_feature
<222> (2)..(2)
<223> The 'Xaa' at location 2 stands for any amino acid.

<400> 38

Lys Xaa Asp
1


<210> 39
<211> 3
<212> PRT
<213> Artificial Sequence

<220>
<223> Retention sequence 5


<220>
<221> misc_feature
<222> (2)..(2)
<223> The 'Xaa' at location 2 stands for any amino acid.

<400>  39

Lys Xaa Glu
1



<210>  40
<211>  4
<212>  PRT
<213>  Artificial Sequence

<220>
<223>  Retention sequence 6

<400>  40

Tyr Gln Arg Leu
1



<210>  41
<211>  20
<212>  PRT
<213>  Artificial Sequence

<220>
<223>  Linker

<400>  41

Gly Gly Gly Gly Ser Gly Gly Gly Gly Ser Gly Gly Gly Gly Ser Gly
1               5                   10                  15


Gly Gly Gly Ser
            20



<210>  42
<211>  120
<212>  PRT
<213>  Artificial Sequence

<220>
<223>  VH

<400>  42

<210>  43
<211>  106
<212>  PRT
<213>  Artificial Sequence

<220>
<223>  VL

<400>  43

<210>  44
<211>  804
<212>  PRT
<213>  Artificial Sequence

<220>
<223>  expression frame

<400>  44

Met Asp Met Arg Val Pro Ala Gln Leu Leu Gly Leu Leu Leu Leu Trp
1               5                   10                  15

Leu Arg Gly Ala Arg Cys Asp Ile Gln Met Thr Gln Ser Pro Ser Ser
            20                  25                  30

Leu Ser Ala Ser Val Gly Asp Arg Val Thr Ile Thr Cys Arg Ala Ser
        35                  40                  45

Gln Asp Ile Ser Lys Tyr Leu Asn Trp Tyr Gln Gln Lys Pro Gly Lys
    50                  55                  60

Ala Pro Lys Leu Leu Ile Tyr His Thr Ser Arg Leu His Ser Gly Val
65                  70                  75                  80

Pro Ser Arg Phe Ser Gly Ser Gly Ser Gly Thr Asp Phe Thr Leu Thr
                85                  90                  95

Ile Ser Ser Leu Gln Pro Glu Asp Phe Ala Thr Tyr Tyr Cys Gln Gln
            100                 105                 110

Gly Asn Thr Leu Pro Tyr Thr Phe Gly Gln Gly Thr Lys Leu Glu Ile
        115                 120                 125

Lys Gly Ser Thr Ser Gly Gly Gly Ser Gly Gly Gly Ser Gly Gly Gly
    130                 135                 140

Gly Ser Ser Gln Val Thr Leu Lys Glu Ser Gly Pro Ala Leu Val Lys
145                 150                 155                 160

Pro Thr Gln Thr Leu Thr Leu Thr Cys Thr Phe Ser Gly Phe Ser Leu
                165                 170                 175

Ser Asp Tyr Gly Val Ser Trp Ile Arg Gln Pro Pro Gly Lys Ala Leu
            180                 185                 190

Glu Trp Leu Ala Val Ile Trp Gly Ser Glu Thr Thr Tyr Tyr Asn Ser
        195                 200                 205

Ala Leu Lys Ser Arg Leu Thr Ile Ser Lys Asp Thr Ser Lys Asn Gln
    210                 215                 220

Val Val Leu Thr Met Thr Asn Met Asp Pro Val Asp Thr Ala Thr Tyr
225                 230                 235                 240

Tyr Cys Ala Lys His Tyr Tyr Tyr Gly Gly Ser Tyr Ala Met Asp Tyr

245                 250                 255

Trp Gly Gln Gly Thr Leu Val Thr Val Ser Ser Thr Thr Thr Pro Ala
        260                 265                 270

Pro Arg Pro Pro Thr Pro Ala Pro Thr Ile Ala Ser Gln Pro Leu Ser
        275                 280                 285

Leu Arg Pro Glu Ala Cys Arg Pro Ala Ala Gly Gly Ala Val His Thr
    290                 295                 300

Arg Gly Leu Asp Phe Ala Cys Asp Ile Tyr Ile Trp Ala Pro Leu Ala
305                 310                 315                 320

Gly Thr Cys Gly Val Leu Leu Leu Ser Leu Val Ile Thr Leu Tyr Cys
                325                 330                 335

Lys Arg Gly Arg Lys Lys Leu Leu Tyr Ile Phe Lys Gln Pro Phe Met
        340                 345                 350

Arg Pro Val Gln Thr Thr Gln Glu Glu Asp Gly Cys Ser Cys Arg Phe
        355                 360                 365

Pro Glu Glu Glu Glu Gly Gly Cys Glu Leu Arg Val Lys Phe Ser Arg
    370                 375                 380

Ser Ala Asp Ala Pro Ala Tyr Gln Gln Gly Gln Asn Gln Leu Tyr Asn
385                 390                 395                 400

Glu Leu Asn Leu Gly Arg Arg Glu Glu Tyr Asp Val Leu Asp Lys Arg
                405                 410                 415

Arg Gly Arg Asp Pro Glu Met Gly Gly Lys Pro Arg Arg Lys Asn Pro
        420                 425                 430

Gln Glu Gly Leu Tyr Asn Glu Leu Gln Lys Asp Lys Met Ala Glu Ala
        435                 440                 445

Tyr Ser Glu Ile Gly Met Lys Gly Glu Arg Arg Arg Gly Lys Gly His
    450                 455                 460

Asp Gly Leu Tyr Gln Gly Leu Ser Thr Ala Thr Lys Asp Thr Tyr Asp
465                 470                 475                 480

Ala Leu His Met Gln Ala Leu Pro Pro Arg Gly Ser Gly Val Lys Gln
                485                 490                 495

Thr Leu Asn Phe Asp Leu Leu Lys Leu Ala Gly Asp Val Glu Ser Asn
            500                 505                 510

Pro Gly Pro Met Asp Met Arg Val Pro Ala Gln Leu Leu Gly Leu Leu
        515                 520                 525

Leu Leu Trp Leu Arg Gly Ala Arg Cys Asp Ile Gln Met Thr Gln Ser
    530                 535                 540

Pro Ser Ser Leu Ser Ala Ser Val Gly Asp Arg Val Thr Ile Thr Cys
545                 550                 555                 560

Ser Ala Ser Ser Ser Val Ser Tyr Met Asn Trp Tyr Gln Gln Lys Pro
                565                 570                 575

Gly Lys Ala Pro Lys Arg Leu Ile Tyr Asp Thr Ser Lys Leu Ala Ser
            580                 585                 590

Gly Val Pro Ser Arg Phe Ser Gly Ser Gly Ser Gly Thr Asp Phe Thr
        595                 600                 605

Leu Thr Ile Ser Ser Leu Gln Pro Glu Asp Phe Ala Thr Tyr Tyr Cys
    610                 615                 620

Gln Gln Trp Ser Ser Asn Pro Pro Thr Phe Gly Gly Gly Thr Lys Val
625                 630                 635                 640

Glu Ile Lys Gly Gly Gly Gly Ser Gly Gly Gly Gly Ser Gly Gly Gly
                645                 650                 655

Gly Ser Gln Val Gln Leu Val Gln Ser Gly Ala Glu Val Lys Lys Pro
            660                 665                 670

Gly Ala Ser Val Lys Val Ser Cys Lys Ala Ser Gly Tyr Thr Phe Ile
        675                 680                 685

Ser Tyr Thr Met His Trp Val Arg Gln Ala Pro Gly Gln Gly Leu Glu
    690                 695                 700

Trp Met Gly Tyr Ile Asn Pro Arg Ser Gly Tyr Thr His Tyr Asn Gln
705                 710                 715                 720

Lys Leu Lys Asp Lys Ala Thr Leu Thr Ala Asp Lys Ser Ala Ser Thr
                725                 730                 735

Ala Tyr Met Glu Leu Ser Ser Leu Arg Ser Glu Asp Thr Ala Val Tyr
            740                 745                 750

Tyr Cys Ala Arg Ser Ala Tyr Tyr Asp Tyr Asp Gly Phe Ala Tyr Trp
        755                 760                 765

Gly Gln Gly Thr Leu Val Thr Val Ser Ser Gly Gly Gly Gly Ser Gly
    770                 775                 780

Gly Gly Gly Ser Gly Gly Gly Gly Ser Gly Gly Gly Gly Ser Ala Glu
785                 790                 795                 800

Lys Asp Glu Leu

<210>  45
<211>  107
<212>  PRT
<213>  Artificial Sequence

<220>
<223>  UCHT1-VL

<400>  45

<210>  46
<211>  122
<212>  PRT
<213>  Artificial Sequence

<220>
<223>  UCHT1-VH

<400>  46

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Thr Met Asn Trp Val Arg Gln Ala Pro Gly Lys Gly Leu Glu Trp Val
        35                  40                  45

Ala Leu Ile Asn Pro Tyr Lys Gly Val Thr Thr Tyr Ala Asp Ser Val
    50                  55                  60

Lys Gly Arg Phe Thr Ile Ser Val Asp Lys Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Gln Gly Thr Leu Val Thr Val Ser Ser
        115                 120


<210>  47
<211>  106
<212>  PRT
<213>  Artificial Sequence

<220>
<223>  OKT3-VL

<400>  47

<210>  48
<211>  119
<212>  PRT
<213>  Artificial Sequence

<220>
<223>  OKT3-VH

<400>  48

Gln Val Gln Leu Val Gln Ser Gly Gly Gly Val Val Gln Pro Gly Arg
1               5                   10                  15

Ser Leu Arg Leu Ser Cys Lys Ala Ser Gly Tyr Thr Phe Thr Arg Tyr
            20                  25                  30

Thr Met His Trp Val Arg Gln Ala Pro Gly Lys Gly Leu Glu Trp Ile
        35                  40                  45

Gly Tyr Ile Asn Pro Ser Arg Gly Tyr Thr Asn Tyr Asn Gln Lys Val
    50                  55                  60

Lys Asp Arg Phe Thr Ile Ser Arg Asp Asn Ser Lys Asn Thr Ala Phe
65                  70                  75                  80

Leu Gln Met Asp Ser Leu Arg Pro Glu Asp Thr Gly Val Tyr Phe Cys
                85                  90                  95

Ala Arg Tyr Tyr Asp Asp His Tyr Cys Leu Asp Tyr Trp Gly Gln Gly
            100                 105                 110

Thr Leu Val Thr Val Ser Ser
        115

<210>  49
<211>  21
<212>  DNA
<213>  Artificial Sequence

<220>
<223>  Primer 1

<400>  49
tcaagcctca gacagtggtt c                                                21

<210>  50
<211>  24
<212>  DNA
<213>  Artificial Sequence

<220>
<223>  Primer 2

<400>  50
ccagtcaatc tttcacaaat tttg                                              24


<210>  51
<211>  2415
<212>  DNA
<213>  Artificial Sequence

<220>
<223>  nucleic acid

<400>  51
atggatatga gggtgcctgc ccagctgctg ggcctgctgc tgctgtggct gaggggcgct        60

aggtgtgaca tccagatgac ccagagccct tcctccctga gcgcctccgt gggcgataga       120

gtgacaatca catgtagagc ctcccaggac atcagcaagt acctgaactg gtaccagcag       180

aagcccggca aggcccccaa gctgctgatc taccacacct ccagactgca cagcggcgtg       240

cctagcaggt tcagcggctc cggcagcggc accgacttta cactgaccat cagctccctg       300

cagcctgagg atttcgccac ctactactgt cagcagggca atacactgcc ctacaccttt       360

ggccagggca ccaagctgga gatcaaggga tccaccagcg gcggaggaag cggcggaggt       420

agcggaggag gcggaagctc ccaggtgaca ctgaaggaga gcggccctgc cctggtgaag       480

cctacacaga cactgacact gacgtgtacc ttctccggct tcagcctgtc cgattacggc       540

gtgagctgga tcagacagcc tcctggcaag gccctggagt ggctggccgt gatctggggc       600

agcgagacca cctactacaa ttccgccctg aagagcaggc tgaccatctc caaggacacc       660

tccaagaacc aggtggtgct gaccatgacc aatatggatc ctgtggacac cgccacatac       720

tactgtgcca agcactacta ctacggcggc agctacgcca tggattactg gggccagggc       780

accctggtga ccgtgagctc caccacgacg ccagcgccgc gaccaccaac accggcgccc       840

accatcgcgt cgcagcccct gtccctgcgc ccagaggcgt gccggccagc ggcggggggc       900

gcagtgcaca cgagggggct ggacttcgcc tgtgatatct acatctgggc gcccttggcc       960

gggacttgtg gggtccttct cctgtcactg gttatcaccc tttactgcaa acggggcaga      1020

aagaaactcc tgtatatatt caaacaacca tttatgagac cagtacaaac tactcaagag      1080

gaagatggct gtagctgccg atttccagaa gaagaagaag gaggatgtga actgagagtg      1140

aagttcagca ggagcgcaga cgcccccgcg taccagcagg gccagaacca gctctataac      1200

gagctcaatc taggacgaag agaggagtac gatgttttgg acaagagacg tggccgggac      1260

cctgagatgg ggggaaagcc gagaaggaag aaccctcagg aaggcctgta caatgaactg 1320

cagaaagata agatggcgga ggcctacagt gagattggga tgaaaggcga gcgccggagg 1380

ggcaaggggc acgatggcct ttaccagggt ctcagtacag ccaccaagga cacctacgac 1440

gcccttcaca tgcaggccct gccccctcgc ggctccggag taaagcaaac actgaacttt 1500

gaccttctca agttggctgg agacgttgag tccaatcctg ggcccatgga catgagagtg 1560

cccgctcaac tgctgggact gctgctgctt tggctgagag gcgccagatg cgacatccag 1620

atgacacaga gccctagcag cctgtctgcc agcgtgggag acagagtgac catcacatgt 1680

agcgccagca gcagcgtgtc ctacatgaac tggtatcagc agaagcccgg caaggcccct 1740

aagcggctga tctacgatac aagcaagctg gcctctggcg tgcccagcag attttctggc 1800

tctggcagcg gcaccgactt caccctgacc atatctagcc tgcagcctga ggacttcgcc 1860

acctactact gccagcagtg gtccagcaat cctcctacct ttggcggagg caccaaggtg 1920

gaaatcaaag gcggcggagg aagcggaggc ggaggatctg gtggtggtgg atctcaggtt 1980

cagctggtgc agtctggcgc cgaagtgaaa aaacctggcg cctccgtgaa ggtgtcctgc 2040

aaggctagcg gctacacctt catcagctac accatgcact gggtccgaca ggcccctgga 2100

caaggacttg agtggatggg ctacatcaac cccagaagcg gctataccca ctacaaccag 2160

aagctgaagg acaaggccac actgaccgcc gataagagcg cctctaccgc ctatatggaa 2220

ctgagcagtc tgcggagcga ggacaccgcc gtgtactact gtgccagaag cgcctactac 2280

gactacgacg gctttgccta ttggggccag ggcacactgg tcacagttag ttctggtggc 2340

ggcggttctg gcggtggcgg cagtggtggt ggcggatcag gcggaggcgg atctgccgag 2400

aaggatgaac tgtga 2415

## Description

### TECHNICAL FIELD

The invention belongs to the field of cellular immunotherapy, in particular to engineered T cells, preparation thereof and use thereof.

### BACKGROUND

With the development of tumor therapy, chimeric antigen receptor-T (CAR-T) immunotherapy has gradually become a great deal of attention. CAR expressed by CAR-T cells generally contain an extracellular antigen binding domain, a transmembrane domain, and an intracellular signaling domain. Usually CAR-T cells are expanded from autologous T cells transduced with a CAR gene of a patient, and are finally reinfused into said patient. CAR-T cells can efficiently recognize tumor antigens, elicit specific antitumor immune responses without the limitation of major histocompatibility complex (MHC). Currently, the U.S. FDA has approved two autologous Car-T cell products, Kymriah from Novartis and YesCAR-Ta from Kate for the treatment of refractory relapsed non Hodgkin's lymphoma and acute lymphoblastic leukemia, respectively. Relevant clinical trials have proven that CAR-T is extremely antitumor potential as a fully personalized live cell drug (Maude S L, Laetsch T W, Buechner J, et al. Tisagenlecleucel in children and young adults with B-cell lymphoblastic leukemia[J]. New England Journal of Medicine, 2018, 378(5): 439-448; Park J H, Rivière I, Gonen M, et al. Long-term follow-up of CD19 CAR therapy in acute lymphoblastic leukemia[J]. New England Journal of Medicine, 2018, 378(5): 449-459; Schuster S J, Svoboda J, Chong E A, et al. Chimeric antigen receptor T cells in refractory B-cell lymphomas[J]. New England Journal of Medicine, 2017, 377(26): 2545-2554).

The preparation and reinfusion strategies of the above two CAR-T drugs are to collect the patient's autologous peripheral blood, isolate and obtain T cells, integrate the coding gene of CAR into the genome of T cells through viral vectors, then do expansion culture, and finally be reinfused into the patient. This fully individualized CAR-T cell production not only has a long production cycle and high cost, but also brings many uncertainties to reinfusion therapy. For example, failure of CAR-T cell preparation due to insufficient numbers or malfunctioning of the isolated T cells, rapid disease progression of the patient during cell preparation with loss of the therapeutic window, etc. Therefore, a common expectation in the CAR-T field is that of healthy donor derived off-the-shelf allogeneic CAR-T cells to effectively avoid or address the aforementioned production and treatment-related issues and to greatly reduce production costs.

Preparation of allogeneic CAR-T cells, one major safety concern first needs to be addressed, namely graft-versus-host disease (GVHD). GVHD is an immune rejection against the recipient's normal tissues and organs induced by the recognition of host allotypic tissue antigens by alloreactive T cells in the graft and is lethal when severe. GVHD is mediated through the T cell receptor (TCR) on the surface of allogeneic T cells. TCR is a characteristic signature on the surface of all mature T cells. TCR binds and assembles intracellularly with multiple CD3 subunits in endoplasmic reticulum (ER) through noncovalent bonds, finally present as a TCR-CD3 antigen recognition complex on the cell surface.

As early as 2012, Provasi *et al.* reported using gene editing technology to try to solve the problem of graft-versus-host reaction of allogeneic T cells (Provasi E, Genovese P, Lombardo A, et al. Editing T cell specificity towards leukemia by zinc finger nucleases and lentiviral gene transfer[J]. Nature medicine, 2012, 18(5): 807). They abrogate GVHD response by specifically knocking out TCR genes in donor T cells through zinc-finger nucleases (ZFN), which results in failure in forming TCR/CD3 complex and inability of T cells to recognize host antigens. Since then, a variety of other novel gene editing technologies, such as transcription activator-like effector nucleases (TALEN) and clustered regularly interspaced short palindromic repeats (CRISPR) are reported to succeed at the laboratory level (Berdien B, Mock U, Atanackovic D, et al. TALEN-mediated editing of endogenous T-cell receptors facilitates efficient reprogramming of T lymphocytes by lentiviral gene transfer[J]. Gene therapy, 2014, 21(6): 539; Ren J, Zhao Y. Advancing chimeric antigen receptor T cell therapy with CRISPR/Cas9[J]. Protein & cell, 2017, 8(9): 634-643). To date, however, only a very limited number of clinical cases of allogeneic CAR-T have been reported (Qasim W, Zhan H, Samarasinghe S, et al. Molecular remission of infant B-ALL after infusion of universal TALEN gene-edited CAR T cells[J]. Science translational medicine, 2017, 9(374): eaaj2013). The slow progress of gene editing based allogeneic CAR-T clinical research may be mainly due to the safety hazards of gene editing (e.g., off targeting leading to erroneous gene editing), the potential for gene editing to reduce in vivo persistence of T cells, and the hurdles encountered in production preparation (e.g., extensive process steps, greater damage to T cells, etc.).

Non-gene editing based allogeneic CAR-T technologies have similarly gained close attention from industry. This technique was derived from a report in 1995 in which the authors showed that intracellular expression of a specific antibody carrying an endoplasmic reticulum retention signal allows efficient retention of the IL-2 receptor protein within the endoplasmic reticulum with almost no presence the cell surface, which achieves a loss of function similar to gene editing (Richardson J H, Sodroski J G, Waldmann T A, et al. Phenotypic knockout of the high-affinity human interleukin 2 receptor by intracellular single-chain antibodies against the alpha subunit of the receptor[J]. Proceedings of the National Academy of Sciences, 1995, 92(8): 3137-3141). This technique has recently been borrowed to find polypeptides that can efficiently downregulate the surface TCR/CD3 complex and whether there are universal, such as which protein in the TCR/CD3 complex or its epitope is most efficiently recognized by the antibody included in the polypeptide. But the results show that antibodies to different targets, different antibody clones to the same target, different ER retention signals, different linkers, and other factors may affect the activity of these antibody sequences to downregulate the surface TCR/CD3 complex; active antibody sequences in turn lack robust down regulatory activity against different donor T cells (Kamiya T, Wong D, Png Y T, et al. A novel method to generate T-cell receptor-deficient chimeric antigen receptor T cells[J]. Blood advances, 2018, 2(5): 517-528; WO2019032916A1; US20180086831A1).

CAR-T cells with downregulated or absent surface TCR/CD3 complex may also improve the efficacy of current autologous CAR-T cell therapies. It is possible that the TCR/CD3 complex that recognizes the patient's own tumor is present on CAR-T cells prepared from the patient's autologous T cells, and simultaneous activation of the TCR/CD3 complex with CAR is known to trigger exhaustion of CD8+ T cells, thereby affecting the therapeutic efficacy of CAR-T cells (Yang Y, Kohler M E, Chien C D, et al. TCR engagement negatively affects CD8 but not CD4 CAR T cell expansion and leukemic clearance[J]. Science translational medicine, 2017, 9(417): eaag1209).

There is a critical need in the field for a method and cells that can efficiently downregulate the surface TCR/CD3 complex from different donor sources of T cells. There is a critical need in the field for a method and cells that can target T cells of different donor origins, i.e., express therapeutically useful proteins, and at the same time efficiently downregulate their surface TCR/CD3 complex.

### SUMMARY OF INVENTION

To this end, the description provides reagents, methods, and cells that efficiently downregulate the TCR/CD3 complex on cell surface.

In one aspect, the description provides an engineered T cell, wherein the T cell expresses a polypeptide that downregulates the cell surface TCR/CD3 complex, wherein the polypeptide comprises a heavy chain variable region (VH) having an amino acid sequence of SEQ ID No: 1 or more than 95% identical to SEQ ID No: 1, and a light chain variable region (VL) having an amino acid sequence of SEQ ID No: 2 or more than 95% identical to SEQ ID No: 2.

In one or more embodiments, the VH of the polypeptide that downregulates the cell surface TCR/CD3 complex comprises HCDR2: X₁X₂X₃X₄SGYT (where X₁, X₂, X₃, X₄ are any amino acid) having a sequence as shown in SEQ ID No: 9.

In one or more embodiments, the VH of the polypeptide that downregulates the cell surface TCR/CD3 complex comprises HCDR3: ARSX₅X₆X₇DYDGFAY (wherein X₅, X₆, X₇ are any amino acid) having a sequence as shown in SEQ ID No: 13.

In one or more embodiments, the VL of the polypeptide that downregulates the cell surface TCR/CD3 complex comprises LCDR3: QQWX₈X₉X₁₀X₁₁X₁₂T (wherein X₈, X₉, X₁₀, X₁₁, X₁₂ are any amino acid) having a sequence as shown in SEQ ID No: 15.

In one or more embodiments, X₁ is serine, isoleucine, alanine, valine, leucine, isoleucine, proline, phenylalanine, methionine, tryptophan, threonine, glycine, asparagine, glutamine, tyrosine, or cysteine.

In one or more embodiments, X₂ is threonine, asparagine, glycine, glutamine, serine, tyrosine, cysteine, valine, or isoleucine.

In one or more embodiments, X₃ is proline, alanine, valine, leucine, isoleucine, phenylalanine, methionine, tryptophan, glycine, asparagine, glutamine, serine, threonine, tyrosine, or cysteine.

In one or more embodiments, X₄ is arginine, lysine, histidine, alanine, valine, leucine, isoleucine, proline, phenylalanine, methionine, or tryptophan.

In one or more embodiments, X₅ is alanine, glycine, glutamine, serine, threonine, tyrosine, cysteine, lysine, arginine, or histidine.

In one or more embodiments, X₆ is tyrosine, alanine, valine, leucine, isoleucine, phenylalanine, methionine, tryptophan, aspartic acid or glutamic acid.

In one or more embodiments, X₇ is tyrosine, lysine, histidine, glycine, asparagine, glutamine, serine, threonine, tyrosine, or cysteine.

In one or more embodiments, X₈ is serine, alanine, glycine, asparagine, glutamine, threonine, tyrosine, cysteine, alanine, valine, leucine, isoleucine, proline, phenylalanine, methionine, tryptophan, lysine, arginine, or histidine.

In one or more embodiments, X₉ is threonine, serine, glycine, asparagine, glutamine, tyrosine, cysteine, lysine, arginine, histidine, phenylalanine, tryptophan, alanine, valine, leucine, isoleucine, proline, methionine, aspartic acid, or glutamic acid.

In one or more embodiments, X₁₀ is glutamine, asparagine, glycine, glutamine, serine, threonine, tyrosine, cysteine, valine, isoleucine, alanine, leucine, proline, phenylalanine, methionine, tryptophan, lysine, arginine or histidine.

In one or more embodiments, X₁₁ is proline, alanine, valine, leucine, isoleucine, phenylalanine, methionine, tryptophan, threonine, isoleucine, lysine, arginine, histidine, tyrosine, tryptophan, glycine, asparagine, glutamine, serine, or cysteine.

In one or more embodiments, X₁₂ is glycine, asparagine, glutamine, serine, threonine, tyrosine, cysteine, alanine, valine, leucine, isoleucine, proline, phenylalanine, methionine, tryptophan.

In one or more embodiments, LCDR3 is selected from the group consisting of SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34.

In one or more embodiments, HCDR2 is selected from the group consisting of SEQ ID No: 10, SEQ ID No: 11, SEQ ID No: 12.

In one or more embodiments, HCDR3 is SEQ ID No: 14.

In one or more embodiments, the amino acid sequence of the heavy chain variable region is selected from the group consisting of: (1) SEQ ID No: 42, (2) a variant of SEQ ID No: 42 that comprises said HCDR2 and/or HCDR3, (3) a sequence more than 95% identical to (1) or (2), and/or, the amino acid sequence of the light chain variable region is selected from the group consisting of: (1) SEQ ID No: 43, (2) a variant of SEQ ID No: 43 that comprises said LCDR3; (3) a sequences more than 95% identical to (1) or (2). In one or more embodiments, HCDR2, HCDR3, and LCDR3 are as described herein.

In one or more embodiments, the polypeptide is a single chain antibody.

In one or more embodiments, the polypeptide that downregulates the cell surface TCR/CD3 complex comprises a localization sequence and optionally a signal peptide.

In one or more embodiments, the localization sequence is selected from the group consisting of endoplasmic reticulum retention sequence, Golgi retention sequence, E3 ubiquitin ligase binding sequence, proteasome localization sequence, or lysosome localization sequence; preferably, the endoplasmic reticulum retention sequence comprises or consists of any one of SEQ ID Nos: 35-40, where X is any amino acid.

In one or more embodiments, there is a linker is included between the localization sequence and the antibody, wherein the linker is as shown in SEQ ID No: 41.

In one or more embodiments, the signal peptide is as shown in positions 516-537 of SEQ ID No: 44.

In one or more embodiments, the T cells further express a therapeutic protein; preferably, the therapeutic protein is a chimeric antigen receptor.

In one or more embodiments, the T cells contain an expression frame of the therapeutic protein and an expression frame of a polypeptide that downregulates the cell surface TCR/CD3 complex, or the coding sequence for the therapeutic protein and the polypeptide that downregulates the cell surface TCR/CD3 complex are in the same expression frame.

In embodiments wherein the coding sequence of the therapeutic protein and the coding sequence of the polypeptide that downregulates the cell surface TCR/CD3 complex are in the same expression frame, a linker sequence is included between the coding sequence of the therapeutic protein and the coding sequence of the polypeptide that downregulates the cell surface TCR/CD3 complex, and the linker sequence enables expression of multiple cistrons on a single vector.

In one or more embodiments, the linker sequence is the coding sequence of 2A peptide.

In one or more embodiments, the 2A peptide comprises F2A, P2A, or T2A peptide.

In one or more embodiments, the chimeric antigen receptor contains, from the N-terminus to the C-terminus: a single chain antibody to an tumor antigen, a hinge region, a transmembrane region, and an intracellular region.

In one or more embodiments, the chimeric antigen receptor specifically binds to one or more of the following tumor antigens: EGFRvIII, mesothelin, GD2, Tn antigen, sTn antigen, Tn-O-glycopeptide, sTn-O-glycopeptide, PSMA, CD97, TAG72, CD44v6, CEA, EPCAM, KIT, IL-13Ra2, leguman, GD3, CD171, IL-11Ra, PSCA, MAD-CT-1, MAD-CT-2, VEGFR2, LewisY, CD24, PDGFR-β, SSEA-4, folate receptor α, ERBB, Her2/neu, MUC1, EGFR, NCAM, ephrin B2, CAIX, LMP2, sLE, HMWMAA, o-acetyl-GD2, folate receptor β, TEM1/CD248, TEM7R, FAP, Podoplanin, HPV E6 or E7, ML-IAP, CLDN6, TSHR, GPRC5D, ALK, polysialic acid, Fos-associated antigen, neutrophil elastase, TRP-2, CYP1B1, sperm protein 17, β HCG, AFP, thyroglobulin, PLAC1, globoH, RAGE1, MN-CA IX, human telomerase reverse transcriptase, intestinal carboxylesterase, mut hsp 70-2, NA-17, NY-BR-1, UPK2, HAVCR1, ADRB3, PANX3, NY-ESO-1, GPR20, Ly6k, OR51E2, TARP, GFRα4 and a polypeptide fragment of any of these antigens presented on the MHC, as well as CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79a, CD79b, CD80, CD81, CD82, CD83, CD84, CD85, CD86, CD123, CD135, CD138, CD179, CD269, Flt3, ROR1, BCMA, FcRn5, FcRn2, CS-1, CXCR4, CXCR5, CXCR7, IL-7/3R, IL7/4/3R and IL4R.

In one or more embodiments, the hinge region is selected from the group consisting of CD8 α hinge region, IgG1 Fc CH2CH3 hinge region, IgD hinge region, CD28 extracellular hinge region, IgG4 Fc CH2CH3 hinge region, and CD4 extracellular hinge region.

In one or more embodiments, the transmembrane region is selected from one or more of CD28 transmembrane region, CD8 transmembrane region, CD3ζ transmembrane region, CD134 transmembrane region, CD137 transmembrane region, ICOS transmembrane region, and DAP10 transmembrane region.

In one or more embodiments, the intracellular region is selected from an intracellular region of one or more of CD28, CD134/OX40, CD137/4-1BB, LCK, ICOS, DAP10, CD3ζ, and Fc310.

In one or more embodiments, the chimeric antigen receptor comprises a signal peptide.

In one or more embodiments, the single chain antibody to the tumor antigen is an anti-CD 19 single chain antibody.

In one or more embodiments, the hinge region is CD8α hinge region.

In one or more embodiments, the transmembrane region is CD8 transmembrane region.

In one or more embodiments, the intracellular region comprises a 4-1BB intracellular region and human CD3ζ intracellular region.

In one or more embodiments, the light chain variable region sequence of the anti-CD19 single chain antibody comprises or consists of amino acids 23-129 of SEQ ID No: 44.

In one or more embodiments, the heavy chain variable region sequence of the anti-CD19 single chain antibody comprises or consists of amino acids 148-267 of SEQ ID No: 44.

In one or more embodiments, the sequence of the hinge region comprises or consists of amino acids 268-312 of SEQ ID No: 44.

In one or more embodiments, the sequence of the transmembrane region comprises or consists of amino acids 313-336 of SEQ ID No: 44.

In one or more embodiments, the 4-1BB intracellular region comprises or consists of amino acids 337-378 of SEQ ID No: 44; the CD3ζ intracellular region comprises or consists of amino acids 379-490 of SEQ ID No: 44.

In one or more embodiments, the signal peptide of the chimeric antigen receptor comprises or consists of amino acids 1-22 of SEQ ID No: 44.

In another aspect, the description provides an engineered T cell, wherein the T cells express a polypeptide that downregulates the TCR/CD3 complex on the cell surface, and the polypeptide comprises: an antibody or an antigen binding fragment thereof, or a mutant having at least 90% sequence identity with the antibody or antigen binding fragment thereof and retaining antigen binding activity thereof; wherein the antibodies comprise a heavy chain variable region and a light chain variable region,

The heavy chain variable region has the following HCDRs: HCDR1: GYTFISYT as shown in SEQ ID No: 3, HCDR2: X₁X₂X₃X₄SGYT as shown in SEQ ID No: 9, where X₁, X₂, X₃, X₄ are any amino acid, HCDR3: ARSX₅X₆X₇DYDGFAY as shown in SEQ ID No: 13, wherein X₅, X₆, X₇ are any amino acid,

The light chain variable region has the following LCDRs:
LCDR1: SSVSY as shown in SEQ ID No: 6, LCDR2: DTS as shown in SEQ ID No: 7, LCDR3: QQWX₈X₉X₁₀X₁₁X₁₂T as shown in SEQ ID No: 15, wherein X₈, X₉, X₁₀, X₁₁, X₁₂ are any amino acid.

Preferably, X₁-X₁₂ are as described herein.

In one or more embodiments, LCDR3 is selected from the group consisting of SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34. In one or more embodiments, HCDR2 is selected from the group consisting of SEQ ID No: 10, SEQ ID No: 11, SEQ ID No: 12. In one or more embodiments, HCDR3 is SEQ ID No: 14.

In one or more embodiments, the amino acid sequence of the heavy chain variable region is selected from the group consisting of: (1) SEQ ID No: 42, (2) a variant of SEQ ID No: 42 containing said HCDR2 and/or HCDR3, (3) a sequence more than 95% identicial to (1) or (2), and/or the amino acid sequence of the light chain variable region is selected from the group consisting of: (1) SEQ ID No: 43, (2) a variant of SEQ ID No: 43 containing said LCDR3; (3) a sequence more than 95% identicial to (1) or (2).

In one or more embodiments, the antibodies are single chain antibodies.

In one or more embodiments, the polypeptide that downregulates the cell surface TCR/CD3 complex comprises a localization sequence and optionally a signal peptide.

In one or more embodiments, the localization sequence is selected from the group consisting of endoplasmic reticulum retention sequence, Golgi retention sequence, E3 ubiquitin ligase binding sequence, proteasome localization sequence, or lysosome localization sequence. Preferably, the endoplasmic reticulum retention sequence comprises or consists of any one of SEQ ID Nos: 35-40, wherein X is any amino acid.

In one or more embodiments, a linker is included between the localization sequence and the antibody, preferably the linker is as shown in SEQ ID No: 41.

In one or more embodiments, the signal peptide is as shown in positions 516-537 of SEQ ID No: 44.

In one or more embodiments, the T cells further express a therapeutic protein, preferably, the therapeutic protein is a chimeric antigen receptor.

In one or more embodiments, the T cells contain an expression frame of the therapeutic protein and an expression frame of a polypeptide that downregulates the cell surface TCR/CD3 complex, or the coding sequence of the therapeutic protein and the coding sequence of the polypeptide that downregulates the cell surface TCR/CD3 complex are in the same expression frame.

The description also provides an antibody or an antigen binding fragment thereof, wherein the amino acid sequence of HCDR1 of the antibody is GYTFISYT, the amino acid sequence of HCDR2 is X₁X₂X₃X₄SGYT, the amino acid sequence of HCDR3 is ARSX₅X₆X₇DYDGFAY, the amino acid sequence of LCDR1 is SSVSY, the amino acid sequence of LCDR2 is DTS, the amino acid sequence of LCDR3 is QQWX₈X₉X₁₀X₁₁X₁₂T; wherein, X₁-X₁₂ are as claimed in claim 10, and HCDR2 is not INPRSGYT, HCDR3 is not ARSAYYDYDGFAY, and LCDR3 is not QQWSSNPPT.

In one or more embodiments, LCDR3 is selected from the group consisting of SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34. In one or more embodiments, HCDR2 is selected from the group consisting of SEQ ID No: 10, SEQ ID No: 11, SEQ ID No: 12. In one or more embodiments, HCDR3 is SEQ ID No: 14.

In one or more embodiments, the amino acid sequence of the heavy chain variable region of the antibody is as shown in SEQ ID No: 42, but the sequences of its HCDR2 and/or HCDR3 are as shown in claim 10 or 11; the amino acid sequence of the light chain variable region of the antibody is as shown in SEQ ID No: 43, but the sequence of its LCDR3 is as shown in claim 10 or 11.

The description also provides a fusion protein comprising a localization sequence, the polypeptide described herein that downregulates the cell surface TCR/CD3 complex, and, optionally, a signal peptide.

In one or more embodiments, the signal peptide is as shown in positions 516-537 of SEQ ID No: 44.

In one or more embodiments, a linker is included between the localization sequence and a polypeptide that downregulates the cell surface TCR/CD3 complex. The linkers are preferably as shown in SEQ ID No: 41.

In one or more embodiments, the fusion protein is as shown in positions 516-804 or 538-804 of SEQ ID No: 44.

In another aspect, the description provides a nucleic acid molecule selected from the group consisting of: (1) a polynucleotide containing the coding sequence of a therapeutic protein and the coding sequence of a polypeptide described herein that downregulates the cell surface TCR/CD3 complex; (2) the coding sequence of the fusion protein described herein; (3) the coding sequences of the antibodies or their antigen binding fragments described herein; and (4) a complementary sequence or fragment of (1) or (2) or (3). Preferably, the therapeutic protein is a chimeric antigen receptor.

In one or more embodiments, a linker sequence is included between the coding sequence of the therapeutic protein and the coding sequence of a polypeptide that downregulates the cell surface TCR/CD3 complex, such that multiple cistrons are capable of being expressed on a single vector.

In one or more embodiments, the linker sequence is the coding sequence of 2A peptide.

In one or more embodiments, the 2A peptide comprises F2A, P2A, or T2A peptide.

In one or more embodiments, the features of the therapeutic protein and the polypeptide that downregulates the cell surface TCR/CD3 complex are as described herein.

In another aspect, the description provides a nucleic acid construct comprising the nucleic acid molecules described herein.

In one or more embodiments, the nucleic acid construct contains an expression frame of the therapeutic protein and an expression frame of the polypeptide that downregulates the cell surface TCR/CD3 complex; or the nucleic acid construct is an expression frame wherein the coding sequence of the therapeutic protein and the coding sequence of the polypeptide that downregulates the cell surface TCR/CD3 complex are in this expression frame.

In one or more embodiments, the nucleic acid construct is a cloning vector or expression vector.

In another aspect, the description provides a lentivirus containing a nucleic acid molecule, a nucleic acid construct described herein.

In another aspect, the description provides a host cell comprising, expressing and/or secreting an antibody or an antigen binding fragment thereof described herein, or a fusion protein and optionally a therapeutic protein described herein. The host cells comprise the nucleic acid molecules, nucleic acid constructs, and/or lentiviruses described herein.

In another aspect, the description provides a pharmaceutical composition containing the antibody or an antigen binding fragment thereof, a fusion protein, and optionally a therapeutic protein, a nucleic acid molecule, a nucleic acid construct, a lentivirus, or a cell described herein.

In another aspect, the description provides use of a polypeptide that downregulates the cell surface TCR/CD3 complex or a coding sequence thereof, antibodies or antigen binding fragments thereof, fusion proteins and optionally therapeutic proteins, nucleic acid molecules, nucleic acid constructs, lentiviruses described herein in the preparation of T cells wherein the cell surface TCR is downregulated, or in the preparation of T cells for cancer therapy. Preferably, said polypeptide that downregulates the cell surface TCR/CD3 complex is as described herein.

### DESCRIPTION OF DRAWINGS

FIG. 1. The effect of polypeptides comprising different anti-CD3 antibody sequences on the surface TCR and CD3 protein levels of CAR-T cells.
FIG. 2. The effect of polypeptides comprising different anti-CD3 antibody sequences on the surface TCR and CD3 protein levels of CAR-T cells.
FIG. 3. The effect of polypeptides comprising CTAB1 antibody sequences on the surface TCR and CD3 protein levels of CAR-T cells from different donor origins.
FIG. 4. A polypeptide comprising the CTAB1 antibody sequence downregulates surface TCR and CD3 protein levels of T cells when expressed independently.
FIGS. 5a-5g. The proportion of T cell subsets that coexpress a polypeptide that downregulates cell surface TCR/CD3 complex and a chimeric antigen receptor.
FIG. 6. In vitro expansion of T cells that coexpress a polypeptide that downregulates cell surface TCR/CD3 complex and a chimeric antigen receptor.
FIG. 7. Cytotoxic activity of T cells that coexpress a polypeptide that downregulates cell surface TCR/CD3 complex and a chimeric antigen receptor.
FIG. 8. IFN-γ secretory capacity of T cells that coexpress a polypeptide that downregulates cell surface TCR/CD3 complex and a chimeric antigen receptor.
FIG. 9. Surface marker distribution of cell libraries containing point mutant polypeptides and gating used during cell sorting.
FIG. 10. Exemplary point mutant polypeptides downregulate the activity of TCR and CD3 on the surface of CAR-T cells.
FIGS. 11a-11c. Exemplary point mutant polypeptides does not affect the immune subset distribution of CAR-T cells.
FIG. 12. Expression of an exemplary point mutant polypeptide does not affect in vitro expansion of CAR-T cells.
FIG. 13. Expression of an exemplary point mutant polypeptide does not affect the cytotoxic activity of CAR-T cells.

### EMBODIMENTS

It is understood that within the scope of the description, each of the above technical features of the description and those specifically described below (such as in examples) may all be combined with one another, thereby constituting a preferred technical solution.

Engineered T cells with decreased expression of the cell surface TCR/CD3 complex are prepared by expressing a polypeptide that downregulates the cell surface TCR/CD3 complex. In the T cells prepared by the method of the description, the expression level of the cell surface TCR/CD3 complex is at least 50%, preferably at least 90%, more preferably at least 99%, or completely inhibited, as compared with a control that does not express the polypeptide.

Herein, the polypeptide that downregulates the cell surface TCR/CD3 complex comprises antibodies or antigen binding fragments thereof. An "antibody" refers to a polypeptide comprising an immunoglobulin sequence element sufficient to enable its specific binding to a target antigen. As is known in the art, a native intact antibody is a tetramer comprising two identical heavy chain polypeptides and two identical light chain polypeptides. Each heavy chain contains at least four domains: variable domain VH at the amino terminus, constant domain CHI, constant domain CH2, and constant domain CH3 at the carboxy terminus. Each light chain contains two domains: the variable domain VL at the amino terminus and the constant domain CL at the carboxy terminus. Each variable domain contains three "complementarity determining regions": CDR1, CDR2, and CDR3, and four "framework" regions: FR1, FR2, FR3, and FR4. An "antigen binding fragment" refers to a fragment in an antibody that can specifically bind to a target antigen. Antibodies or antigen binding fragments thereof include Fab fragments, Fab' fragments, F(ab')2 fragments, Fd' fragments, Fd fragments, Fv fragments, disulfide bonded Fv fragments, single chain antibodies (scFv), isolated CDR or CDR groups, polypeptide-Fc fusions, single domain antibodies, camel antibodies, masking antibodies, small module immunodrugs, bifunctional antibodies, nanobodies, Humabody antibodies. Single chain antibodies are antibodies composed of antibody heavy and light chain variable regions linked by a short peptide (linker).

In preferred embodiments, the polypeptide that downregulates the cell surface TCR/CD3 complex is an anti-CD3 antibody that specifically binds to CD3, more preferably an anti-CD3 single chain antibody (scFv). In one or more embodiments, the anti-CD3 antibody is selected from the group consisting of UCHT1, OKT3, CTAB1, CTAB2, CTAB4 or CTAB3. Preferably, anti-CD3 single chain antibodies are derived from these anti-CD3 antibodies. Exemplarily, the VL and VH sequences of the UCHT1 scFv contain or consist of SEQ ID Nos: 45 and 46, respectively; the VL and VH sequences of the OKT3 scFv contain or consist of SEQ ID Nos: 47 and 48, respectively. In preferred embodiments, the anti-CD3 single chain antibody is derived from CTAB1.

In a preferred embodiment, the anti-CD3 antibody comprises the heavy chain variable region having the following HCDRs: HCDR1: GYTFISYT as shown in SEQ ID No: 3, HCDR2: X₁X₂X₃X₄SGYT as shown in SEQ ID No: 9, wherein X₁, X₂, X₃, X₄ are any amino acid, HCDR3: ARSX₅X₆X₇DYDGFAY as shown in SEQ ID No: 13, wherein X₅, X₆, X₇ are any amino acid; the light chain variable region having the following LCDRs: LCDR1: SSVSY as shown in SEQ ID No: 6, LCDR2: DTS as shown in SEQ ID No: 7, LCDR3: QQWX₈X₉X₁₀X₁₁X₁₂T as shown in SEQ ID No: 15, wherein X₈, X₉, X₁₀, X₁₁, X₁₂ are any amino acid.

In one or more embodiments, X₁ is serine, isoleucine, alanine, valine, leucine, isoleucine, proline, phenylalanine, methionine, tryptophan, threonine, glycine, asparagine, glutamine, tyrosine, or cysteine. In one or more embodiments, X₂ is threonine, asparagine, glycine, glutamine, serine, tyrosine, cysteine, valine, or isoleucine. In one or more embodiments, X₃ is proline, alanine, valine, leucine, isoleucine, phenylalanine, methionine, tryptophan, glycine, asparagine, glutamine, serine, threonine, tyrosine, or cysteine. In one or more embodiments, X₄ is arginine, lysine, histidine, alanine, valine, leucine, isoleucine, proline, phenylalanine, methionine, or tryptophan. In one or more embodiments, X₅ is alanine, glycine, glutamine, serine, threonine, tyrosine, cysteine, lysine, arginine, or histidine. In one or more embodiments, X₆ is tyrosine, alanine, valine, leucine, isoleucine, phenylalanine, methionine, tryptophan, aspartic acid or glutamic acid. In one or more embodiments, X₇ is tyrosine, lysine, histidine, glycine, asparagine, glutamine, serine, threonine, tyrosine, or cysteine. In one or more embodiments, X₈ is serine, alanine, glycine, asparagine, glutamine, threonine, tyrosine, cysteine, alanine, valine, leucine, isoleucine, proline, phenylalanine, methionine, tryptophan, lysine, arginine, or histidine. In one or more embodiments, X₉ is threonine, serine, glycine, asparagine, glutamine, tyrosine, cysteine, lysine, arginine, histidine, phenylalanine, tryptophan, alanine, valine, leucine, isoleucine, proline, methionine, aspartic acid, or glutamic acid. In one or more embodiments, X₁₀ is glutamine, asparagine, glycine, glutamine, serine, threonine, tyrosine, cysteine, valine, isoleucine, alanine, leucine, proline, phenylalanine, methionine, tryptophan, lysine, arginine or histidine. In one or more embodiments, X₁₁ is proline, alanine, valine, leucine, isoleucine, phenylalanine, methionine, tryptophan, threonine, isoleucine, lysine, arginine, histidine, tyrosine, tryptophan, glycine, asparagine, glutamine, serine, or cysteine. In one or more embodiments, X₁₂ is glycine, asparagine, glutamine, serine, threonine, tyrosine, cysteine, alanine, valine, leucine, isoleucine, proline, phenylalanine, methionine, tryptophan.

Preferably, LCDR3 was selected from the group consisting of SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID No: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34. In one or more embodiments, HCDR2 is selected from the group consisting of SEQ ID No: 10, SEQ ID No: 11, SEQ ID No: 12. In one or more embodiments, HCDR3 is SEQ ID No: 14.

In some embodiments, the heavy chain variable region of the polypeptide that downregulates the cell surface TCR/CD3 complex comprises or consists of the sequence as shown in SEQ ID No: 42, or a variant of SEQ ID No: 42 that comprises the following HCDRs: HCDR1: GYTFISYTAS as shown in SEQ ID No: 3, HCDR2: X₁X₂X₃X₄SGYT as shown in SEQ ID No: 9, wherein X₁, X₂, X₃, X₄ are any amino acid, HCDR3: ARSX₅X₆X₇DYDGFAY as shown in SEQ ID No: 13, wherein X₅, X₆, X₇ are any amino acid. In one or more embodiments, X₁ is serine, isoleucine, alanine, valine, leucine, isoleucine, proline, phenylalanine, methionine, tryptophan, threonine, glycine, asparagine, glutamine, tyrosine, or cysteine. In one or more embodiments, X₂ is threonine, asparagine, glycine, glutamine, serine, tyrosine, cysteine, valine, or isoleucine. In one or more embodiments, X₃ is proline, alanine, valine, leucine, isoleucine, phenylalanine, methionine, tryptophan, glycine, asparagine, glutamine, serine, threonine, tyrosine, or cysteine. In one or more embodiments, X₄ is arginine, lysine, histidine, alanine, valine, leucine, isoleucine, proline, phenylalanine, methionine, or tryptophan. In one or more embodiments, X₅ is alanine, glycine, glutamine, serine, threonine, tyrosine, cysteine, lysine, arginine, or histidine. In one or more embodiments, X₆ is tyrosine, alanine, valine, leucine, isoleucine, phenylalanine, methionine, tryptophan, aspartic acid or glutamic acid. In one or more embodiments, X₇ is tyrosine, lysine, histidine, glycine, asparagine, glutamine, serine, threonine, tyrosine, or cysteine.

In one or more embodiments, the sequence of the light chain variable region of the polypeptide that downregulates the cell surface TCR/CD3 complex comprises or consists of the sequence as shown in SEQ ID No: 43 or a variant of SEQ ID No: 43 comprising the following LCDRs: LCDR1: SSVSY as shown in SEQ ID No: 6, LCDR2: DTS as shown in SEQ ID No: 7, LCDR3: QQWX₈X₉X₁₀X₁₁X₁₂T as shown in SEQ ID No: 15, wherein X₈, X₉, X₁₀, X₁₁, X₁₂ are any amino acid. In one or more embodiments, X₈ is serine, alanine, glycine, asparagine, glutamine, threonine, tyrosine, cysteine, alanine, valine, leucine, isoleucine, proline, phenylalanine, methionine, tryptophan, lysine, arginine, or histidine. In one or more embodiments, X₉ is threonine, serine, glycine, asparagine, glutamine, tyrosine, cysteine, lysine, arginine, histidine, phenylalanine, tryptophan, alanine, valine, leucine, isoleucine, proline, methionine, aspartic acid, or glutamic acid. In one or more embodiments, X₁₀ is glutamine, asparagine, glycine, glutamine, serine, threonine, tyrosine, cysteine, valine, isoleucine, alanine, leucine, proline, phenylalanine, methionine, tryptophan, lysine, arginine or histidine. In one or more embodiments, X₁₁ is proline, alanine, valine, leucine, isoleucine, phenylalanine, methionine, tryptophan, threonine, isoleucine, lysine, arginine, histidine, tyrosine, tryptophan, glycine, asparagine, glutamine, serine, or cysteine. In one or more embodiments, X₁₂ is glycine, asparagine, glutamine, serine, threonine, tyrosine, cysteine, alanine, valine, leucine, isoleucine, proline, phenylalanine, methionine, tryptophan.

In some embodiments, the sequence of the heavy chain variable region of the polypeptide that downregulates the cell surface TCR/CD3 complex comprises the sequence as shown in SEQ ID No: 42, but wherein HCDR2 is selected from the group consisting of SEQ ID No: 10, SEQ ID No: 11, SEQ ID No: 12, and/or HCDR3 is SEQ ID No: 14. In one or more embodiments, the sequence of the light chain variable region of the polypeptide that downregulates the cell surface TCR/CD3 complex comprises the sequence as shown in SEQ ID No: 43, but wherein LCDR3 is selected from the group consisting of SEQ ID No: 16, SEQ ID No: 16, SEQ ID No: 17, SEQ ID No: 17, SEQ ID No: 18, SEQ ID No: 19, SEQ ID No: 20, SEQ ID No: 20, SEQ ID No: 21, SEQ ID No: 22, SEQ ID No: 22, SEQ ID No: 23, SEQ ID NO: 24, SEQ ID No: 25, SEQ ID No: 25, SEQ ID No: 26, SEQ ID No: 27, SEQ ID No: 27, SEQ ID No: 28, SEQ ID No No: 28, SEQ ID No: 29, SEQ ID No: 16, SEQ ID No: 16, SEQ ID SEQ ID No: 31 SEQ ID NO:32, SEQ ID NO:33, SEQ ID NO:34.

The light chain variable and heavy chain variable regions of the polypeptide that downregulates the cell surface TCR/CD3 complex described herein can be directly connected or through a linker sequence well known in the art, such as the G- and S-containing linker sequences described previously.

In the present description, the polypeptide that downregulates the cell surface TCR/CD3 complex also include mutants that have at least 70% sequence identity with said antibody (especially the anti-CD3 antibody described herein) or its antigen binding fragment and retain the antigen binding activity of said antibody or antigen binding fragment. The mutants include: amino acid sequences that have at least 70%, at least 80%, preferably at least 85%, preferably at least 90%, preferably at least 95%, preferably at least 97% sequence identity with the reference sequence and retain the biological activity (such as activating T cells, binding to TCR) of the reference sequence. The sequence identity between two aligned sequences can be calculated with, e.g., BLASTp from NCBI. Mutants also include amino acid sequences that have one or several mutations (insertions, deletions, or substitutions) in the reference amino acid sequence while still retaining the biological activity of that reference sequence. Said several mutations usually refer to 1-50, such as 1-20, 1-10, 1-8, 1-5 or 1-3 mutations. The substitution is preferably a conservative substitution. For scFvs, mutations can occur either within CDR regions (including those described previously) or within FR regions, as long as the biological activity of the reference sequence remains after mutation. For example, in the art, conservative substitutions with amino acids of approaching or similar performance generally do not alter the function of a protein or polypeptide. "Amino acids of approaching or similar performance" include, for example, families of amino acid residues with similar side chains, which include amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine), amino acids with nonpolar side chains (e.g., alanine, valine, leucine, isoleucine, proline, phenylalanine, methionine, tryptophan), amino acids with β-branched side chain (e.g., threonine, valine, isoleucine) and amino acids with aromatic side chains (e.g., tyrosine, phenylalanine, tryptophan, histidine). Accordingly, the substitution of one or several sites in the present polypeptide with another amino acid residue from the same side chain class will not substantially affect its activity. In some embodiments, the polypeptide that downregulates the cell surface TCR/CD3 complex is a fusion protein, which also comprises a localization sequence that can direct the polypeptide to subcellular structures. Said localization sequences may be located at the N- or C-terminus of the polypeptide. The subcellular structures include, but are not limited to, the Golgi apparatus or endoplasmic reticulum, proteasomes, cell membranes, or lysosomes. The localization sequences include an endoplasmic reticulum retention sequence, a Golgi retention sequence, an E3 ubiquitin ligase binding sequence, a proteasome localization sequence, or a lysosome localization sequence. The localization sequence can be connected directly to its adjacent polypeptide or can be connected by a linker sequence that is well-known in the art, such as the G- and S-containing linker sequences described previously. In one or more embodiments, there is a sequence of SEQ ID No: 41 between the localization sequence and its adjacent polypeptide, such as an antibody or an antigen binding fragment thereof.

The "endoplasmic reticulum retention sequence" or "endoplasmic reticulum retention signal" described herein is a short peptide signal sequence. The ER retention sequence has corresponding receptors on the membrane of the Golgi apparatus and can be transported back to the ER via reflux vesicles. The endoplasmic reticulum retention sequence applicable to the present description may be an endoplasmic reticulum retention sequence well known in the art. The endoplasmic reticulum retention sequence may be located to the C-terminus of the adjacent polypeptide. The "endoplasmic reticulum retention sequence" or "Golgi retention sequence" includes, but is not limited to, or consists of AEKDEL (SEQ ID No: 35), KDEL (SEQ ID No: 36), KKXX (SEQ ID No: 37), KXD (SEQ ID No: 38), KXE (SEQ ID No: 39) or YQRL (SEQ ID No: 40), wherein X is any amino acid. Proteasome localization sequences are obtained by linking antibody sequences to an E3 ubiquitin ligase targeting domain containing sequence and enabling coexpression of the nucleic acid sequence of E3 ubiquitin ligase protein. E3 ubiquitin ligase proteins bind to the Fc domain of antibodies with high affinity and can recruit ubiquitin-proteosome complexes to degrade molecules (e.g., proteins and peptides) bound to antibodies. E3 ubiquitin ligase protein targeting domain sequences encode amino acid sequences selected from human immunoglobulin G (IgG) constant region (Fc) genes, such as IgG1, IgG2, or IgG4, and are used to form fusion proteins containing antibody and Fc domains. The "proteasome localization sequence" comprises, but is not limited to, PEST motif. The "lysosomal localization sequence" comprises, but is not limited to, KFERQ.

Polypeptides that downregulate the cell surface TCR/CD3 complex may also include signal peptides. The signal peptide can be a membrane protein signal peptide, such as the signal peptide of antibody heavy chain, the signal peptide of antibody light chain, CD8 signal peptide, CD28 signal peptide, and CD4 signal peptide. The amino acid sequence of an exemplary signal peptide may comprise or consist of amino acid residues 516-537 of SEQ ID No: 44.

Therapeutic polypeptides can be further expressed in the engineered T cells. In allogeneic therapy, these T cells effectively avoid graft-versus-host reactions. In the treatment with autologous reinfusion, the T cells prepared by the method of the present description avoid CD8+ T cell exhaustion caused by simultaneous activation of TCR/CD3 complex and CAR, thereby improving therapeutic efficacy.

The "therapeutic protein" or "therapeutic polypeptide" used herein refers to a molecule that exerts a corresponding therapeutic activity after expression in cells (particularly T cells), which may be natural or isolated from a natural environment, or may be a substance of manufacture, including but not limited to: a protein or polypeptide derived from a virus, bacterium, plant, animal, a small molecule active peptide, an antigen, or a fragment thereof (e.g., antigenic epitope, antigenic determinant), an antibody or a fragment thereof (e.g., heavy chain, light chain, Fab, Fv, scFv), an antigen receptor (e.g., chimeric antigen receptor CAR), a fusion protein or a polypeptide, etc.

Accordingly, the description also provides a CAR-T cell comprising a chimeric antigen receptor (CAR) targeting a tumor antigen of interest and a polypeptide that downregulates the cell surface TCR/CD3 complex. The description also provides a CAR-T cell comprising a polynucleotide encoding a chimeric antigen receptor (CAR) targeting a tumor antigen of interest and a polynucleotide encoding a polypeptide that downregulates the cell surface TCR/CD3 complex.

Herein, suitable T cells can be various T cells well known in the art, especially those routinely used in cellular immunotherapy, including but not limited to peripheral blood T lymphocytes, cytotoxic killer T cells, helper T cells, suppressor/regulatory T cells, γδ T cells, cytokine induced killer cells, and tumor infiltrating lymphocytes, among others, and a mixture of any one or more of the above cells. Herein, CAR-T cells refer to T cells that at least express chimeric antigen receptors.

Herein, the chimeric antigen receptor, with the implications known in the art, is an artificially engineered receptor capable of anchoring specific molecules, such as antibodies, that recognize antigens on the surface of tumor cells to immune cells, such as T cells, allowing immune cells to recognize tumor antigens and kill tumor cells. Chimeric antigen receptors suitable for use herein may be various CARs well known in the art. Typically, CARs contain a polypeptide that binds a tumor antigen, a hinge region, a transmembrane region, and one or more intracellular signaling regions.

Polypeptides suitable for binding tumor antigens may be natural polypeptides or synthetic polypeptides; preferably, the synthetic polypeptide is a single chain antibody or Fab fragment. The "tumor antigen" or "tumor surface antigen" described herein include tumor specific antigens and tumor associated antigens expressed on the surface of tumor cells. Tumor specific antigens are specifically expressed on the surface of tumor cells with no expression in normal tissues. Tumor associated antigens are overexpressed on the surface of tumor cells and lowly expressed in normal tissues. Tumor associated antigens include surface antigens of immune cells, such as CD19; antigens involved in growth and differentiation signals; antigens involved in tumor angiogenesis; and tumor stromal and extracellular matrix antigens of tumor supporting structures.

Herein, tumor antigens of interest include, but are not limited to, solid tumor antigens, myeloid tumor antigens, as well as antigens of non-B cell lineage hematologic tumors. Suitable solid tumor antigens include: EGFRvIII, mesothelin, GD2, Tn antigen, sTn antigen, Tn-O-glycopeptide, sTn-O-glycopeptide, PSMA, CD97, TAG72, CD44v6, CEA, EPCAM, KIT, IL-13Ra2, leguman, GD3, CD171, IL-11Ra, PSCA, MAD-CT-1, MAD-CT-2, VEGFR2, LewisY, CD24, PDGFR-β, SSEA-4, folate receptor α, ERBB (such as ERBB2), Her2/neu, MUC1, EGFR, NCAM, ephrin B2, CAIX, LMP2, sLE, HMWMAA, o-acetyl-GD2, folate receptor β, TEM1/CD248, TEM7R, FAP, Podoplanin, HPV E6 or E7, ML-IAP, CLDN6, TSHR, GPRC5D, ALK, polysialic acid, Fos-associated antigen, neutrophil elastase, TRP-2, CYP1B1, sperm protein 17, β HCG, AFP, thyroglobulin, PLAC1, globoH, RAGE1, MN-CA IX, human telomerase reverse transcriptase, intestinal carboxylesterase, mut hsp 70-2, NA-17, NY-BR-1, UPK2, HAVCR1, ADRB3, PANX3, NY-ESO-1, GPR20, Ly6k, OR51E2, TARP, GFRα4 and a polypeptide fragment of any of these antigens presented on the MHC. Suitable B cell antigens include, but are not limited to CD5, CD10, CD19, CD20, CD21, CD22, CD23, CD24, CD25, CD27, CD30, CD34, CD37, CD38, CD40, CD53, CD69, CD72, CD73, CD74, CD75, CD77, CD79a, CD79b, CD80, CD81, CD82, CD83, CD84, CD85, CD86, CD123, CD135, CD138, CD179, CD269, Flt3, ROR1, BCMA, FcRn5, FcRn2, CS-1, CXCR4, CXCR5, CXCR7, IL-7/3R, IL7/4/3R and IL4R.

In preferred embodiments, the polypeptide herein that binds a tumor antigen is a single chain antibody that specifically binds to any of the above tumor antigens. Herein, a single chain antibody (scFv) refers to an antibody fragment with the ability to bind antigen, composed of antibody light chain variable (VL) region amino acid sequences and heavy chain variable (VH) region amino acid sequences linked by a hinge. Single chain antibodies of interest may be derived from the antibodies of interest. Antibodies of interest may be human antibodies, including human mouse chimeric and humanized antibodies. Antibodies can be secretory, or membrane anchored, preferably membrane anchored. Herein, specific binding refers to the reaction between an antibody or its antigen binding fragment and the antigen to which it is directed. In some embodiments, an antibody that specifically binds to an antigen (or an antibody specific for an antigen) means that the antibody binds to that antigen with an affinity (KD) of less than approximately 10⁻⁵ M, e.g., less than approximately 10⁻⁶ M, 10⁻⁷ M, 10⁻⁸ M, 10⁻⁹ M, or 10⁻¹⁰ M or less.

Antibodies to a tumor antigen that are applicable to the description may be derived from single chain antibodies to various tumor antigens well known in the art. Single chain antibodies may contain the heavy and light variable regions of the antibody of interest or consist of heavy and light variable regions and, optionally, a linker. The heavy and light chain variable regions may be linked with a well-known linker. Herein, a linker or hinge is the polypeptide fragment that links between different proteins or polypeptides with the aim of maintaining the attached proteins or polypeptides in their respective spatial conformations in order to maintain the function or activity of the proteins or polypeptides. Exemplary linkers include those containing G and/or S. In general, the linker contains one or more repeated motifs. For example, the motif may be GGGS, GGGGS, SSSSG, GSGSA, and GGSGG. Preferably, these motifs are adjacent in the linker sequence with no amino acid residues inserted between the repeats. The linker sequence may contain 1, 2, 3, 4, or 5 repeated motifs. The length of the linker can be 3-25 amino acid residues, such as 3-15, 5-15, 10-20 amino acid residues. In certain embodiments, the linker sequence is a poly-glycine linker sequence. There is no particular limit to the number of glycines in the linker sequence, which is typically 2-20, e.g., 2-15, 2-10, 2-8. In addition to glycine and serine, the linker may contain other known amino acid residues such as alanine (A), leucine (L), threonine (T), glutamic acid (E), phenylalanine (F), arginine (R), glutamine (Q), and others. The length of the linker is typically 15-20 amino acids. In certain embodiments, the different proteins or polypeptides of the present description are linked by (GGGS)n, wherein n is an integer ranging from 1 to 5. In certain embodiments, the light chain variable and heavy chain variable regions of a single chain antibody of the present description are linked by (GGGS)n, wherein n is an integer ranging from 1 to 5. In certain embodiments, the light chain variable and heavy chain variable regions of a single chain antibody of the present description are linked by GSTSGGGSGGTGGGSS (SEQ ID No: 41).

In embodiments wherein the single chain antibody to a tumor antigen is an anti-CD 19 single chain antibody, the tumor antigen of interest is CD19, and the single chain antibody of interest is a single chain antibody that specifically binds to CD19. In one or more embodiments, the anti-CD 19 single chain antibody is derived from FMC63. Exemplarily, the light chain variable region sequence of the anti-CD 19 single chain antibody comprises or consists of amino acids 23-129 of SEQ ID No: 44; the heavy chain variable region of the anti-CD 19 single chain antibody comprises or consists of amino acids 148-267 of SEQ ID No: 44, wherein the heavy and light variable regions are linked by a G- and S-containing linker sequence.

Other parts contained in CAR, such as the hinge region, transmembrane region, and intracellular signaling region can be the hinge region, transmembrane region, and intracellular signaling region routinely used to construct various types of CARs.

In the description, the hinge region is a region between the heavy chain CH1 and CH2 functional regions of immunoglobulin, which is proline rich and does not form α helices, prone to stretch and some distortion, in favor of the complementary binding between the antigen binding site of the antibody and the antigenic epitope. The hinge region suitable for the CARs of the present description is well known in the art. The hinge region suitable for use herein may be selected from CD8α hinge region, IgG1 Fc CH2CH3 hinge region, IgD hinge region, CD28 extracellular hinge region, IgG4 Fc CH2CH3 hinge region, and CD4 extracellular hinge region. In one or more embodiments, the hinge region is human CD8α hinge region. The human CD8α hinge region may be derived from CD8α polypeptide chains well known in the art. Exemplarily, the sequence of human CD8α hinge region comprises or consists of amino acids 268 - 312 of SEQ ID No: 44.

In the description, the transmembrane region may be selected from one or more of CD28 transmembrane region, CD8 transmembrane region, CD3ζ transmembrane region, CD134 transmembrane region, CD137 transmembrane region, ICOS transmembrane region, and DAP10 transmembrane region. Preferably, the transmembrane region of the chimeric antigen receptor used herein is the CD8 transmembrane region. The amino acid sequence of the exemplary transmembrane region comprises or consists of amino acid residues 313-336 of SEQ ID No: 44.

In the description, the intracellular signaling region can be selected from the intracellular signaling region of any one or more of CD28, CD134/OX40, CD137/4-1BB, LCK, ICOS, DAP10, CD3ζ and Fc310/, preferably 4-1BB intracellular signaling region and CD3ζ intracellular signaling region. The amino acid sequence of the intracellular signaling region exemplified herein is shown as amino acid residues 337-490 of SEQ ID No: 44. In one or more embodiments, the 4-1BB intracellular region comprises or consists of amino acids 337-378 of SEQ ID No: 44; the CD3ζ intracellular region comprises or consists of amino acids 379-490 of SEQ ID No: 44.

Therapeutic proteins such as chimeric antigen receptors may also include signal peptides. Signal peptides are short peptide chains (5-30 amino acids in length) that guide the transfer of newly synthesized proteins into a secretory pathway, which generally refer to the amino acid sequence at the N-terminus of newly synthesized polypeptide chains that serves to direct transmembrane transfer (localization) of proteins. The signal peptide may be a membrane protein signal peptide such as CD8 signal peptide, CD28 signal peptide, and CD4 signal peptide. The amino acid sequence of an exemplary signal peptide may comprise or consist of amino acid residues 1-22 of SEQ ID No: 44 , or comprises or consists of amino acid residues 1-490 of SEQ ID No: 44.

Thus, in one or more embodiments, the CAR contains, from the N-terminus to the C-terminus, optionally a single chain antibody to a tumor antigen, a hinge region, a transmembrane region, one or more intracellular regions. The amino acid sequence of an exemplary chimeric antigen receptor comprises or consists of amino acid residues 23-490 of SEQ ID No: 44.

The above portions of a chimeric antigen receptor, such as a signal peptide, the light and heavy variable regions of a single chain antibody, a hinge region, a transmembrane region, and an intracellular signal region, may be joined directly to one another or by linker sequences well known in the art, such as the G- and S-containing linker sequences described previously. The therapeutic proteins of the present description, as well as polypeptides that downregulate the cell surface TCR/CD3 complex can be linked directly, or by linker sequences such as the G- and S-containing linker sequences described previously. Alternatively, the therapeutic proteins as well as the polypeptides that downregulate the cell surface TCR/CD3 complex also contain linker sequences, such as 2A peptide, which can express multiple polycistrons on a single vector. As is well known in the art, the 2A peptide is a short peptide that can induce ribosome skipping, including F2A, P2A, T2A peptide, etc. In one or more embodiments, the amino acid sequence of the 2A peptide comprises or consists of amino acids 494-515 of SEQ ID No: 44. The 2A peptides can also be linked to the polypeptides on both sides via conventional G- and S-containing linkers.

The present description comprises polynucleotides encoding polypeptides or proteins that downregulate the cell surface TCR/CD3 complex. The polynucleotides of the present description may be in DNA form or RNA form. A DNA form comprises cDNA, genomic DNA, or artificially synthesized DNA. DNA can be single stranded or double stranded. DNA can be either the coding or noncoding strand. The description also includes degenerate variants of a polynucleotide encoding a polypeptide or protein, i.e., a polynucleotide encoding the same amino acid sequence but differing in nucleotide sequence.

The polynucleotides described herein include sequences that have been changed by codon optimization, as long as the amino acid sequence encoded by the polynucleotide is invariant. Codon optimized sequences may show more suitable expressivity for specific species. Methods for codon optimization of polynucleotide sequences are well known in the art.

The polynucleotides of the description may be a coding sequence of a therapeutic protein such as CAR and a coding sequence of a polypeptide that downregulates the cell surface TCR/CD3 complex, or an expression frame of the therapeutic protein and an expression frame of the protein or polypeptide. In the description, the coding sequence refers to the portion of the nucleic acid sequence that directly determines the amino acid sequence of its protein product (e.g., CAR, single chain antibody, hinge region, transmembrane region, intracellular signaling region, or a polypeptide against downregulated cell surface TCR/CD3 complex, etc.). The boundaries of the coding sequence are usually defined by the ribosome binding site immediately upstream of the open reading frame at the 5 'end of the mRNA (for prokaryotic cells) and the transcription termination sequence immediately downstream of the open reading frame at the 3' end of the mRNA. Coding sequences may include, but are not limited to, DNA, cDNA, and recombinant nucleic acid sequences. In the description, the expression frame refers to the complete elements required for expression of the gene of interest, including a promoter, a gene coding sequence, and a PolyA tailing signal sequence. The polynucleotides described herein may be independently two nucleic acid molecules containing the coding sequence of a therapeutic protein and that of a polypeptide that downregulates the cell surface TCR/CD3 complex, respectively, which are the expression frame of a therapeutic protein and the expression frame of the polypeptide, respectively; alternatively, the coding sequence of the therapeutic protein and the coding sequence of the polypeptide that downregulates the cell surface TCR/CD3 complex may be linked with a linker to form one nucleic acid molecule, as if the coding sequence of the therapeutic protein and the coding sequence of the polypeptide are within the same expression frame, or both expression frames may be linked with a suitable linker to form one nucleic acid molecule. In certain embodiments, the polynucleotide of the description is a nucleic acid molecule wherein the coding sequence of the therapeutic protein and the coding sequence of the polypeptide are within the same expression frame, which comprises a promoter, a nucleic acid sequence encoding the therapeutic protein and the polypeptide, and a PolyA tailing signal. In one or more embodiments, the polynucleotide further comprises an optional coding sequence of an endoplasmic reticulum retention sequence.

In certain embodiments, the coding sequence or expression frame is integrated into the genome of a T cell. Thus, in these embodiments, an expression frame comprising encoding the therapeutic proteins and the polypeptides described herein is stably integrated in the genome of the T cells described herein.

In one or more embodiments, the polynucleotides of the description contain the coding sequences of the following sequences: a single chain antibody to a tumor antigen, human CD8α hinge region, human CD8 transmembrane region, 4-1BB intracellular region, human CD3ζ intracellular region, F2A peptide, antibody polypeptide, and endoplasmic reticulum retention sequence. In one or more embodiments, the polynucleotide comprises or consists of the following nucleic acid sequences: (1) a polynucleotide comprising the coding sequence of a therapeutic protein described herein and the coding sequence for a polypeptide that downregulates the cell surface TCR/CD3 complex described herein; (2) the coding sequence of the fusion protein described herein; and (3) a complementary sequence or fragment of (1) or (2). In one or more embodiments, the polynucleotide comprises or consists of SEQ ID No: 51.

The description also relates to a nucleic acid construct containing the polynucleotides described herein, and one or more regulatory sequences operationally linked to such sequences. The polynucleotides described herein can be manipulated in a variety of ways to ensure the expression of polypeptides that downregulate the cell surface TCR/CD3 complex or proteins (therapeutic proteins and/or downregulated polypeptides). Nucleic acid constructs can be manipulated according to the difference or requirements of the expression vector prior to insertion of the nucleic acid construct into a vector. Techniques that utilize recombinant DNA methods to change polynucleotide sequences are known in the art.

Regulatory sequences can be suitable promoter sequences. Promoter sequences are often operationally linked to the coding sequence of the protein to be expressed. A promoter may be any nucleotide sequence showing transcriptional activity in a selected host cell, including mutated, truncated, and hybrid promoters, and may be obtained from a gene encoding an extracellular or intracellular polypeptide that is homologous or heterologous to that host cell. Regulatory sequences may also be suitable transcription terminator sequences, sequences recognized by host cells to terminate transcription. The terminator sequence was operationally linked to the 3' end of the nucleotide sequence encoding the polypeptide. Any terminator that is functional in the selected host cell can be used in the present description. Regulatory sequences can also be suitable leader sequences, untranslated regions of mRNAs important for host cell translation. The leader sequence is operationally linked to the 5' end of the nucleotide sequence encoding the polypeptide. Any terminator that is functional in the selected host cell can be used in the present description.

In certain embodiments, the nucleic acid construct is a vector. The vector may be a cloning vector, an expression vector, or a homologous recombination vector. The polynucleotides of the present description can be cloned into many types of vectors, e.g., plasmids, phagemids, phage derivatives, animal viruses, and cosmids. Cloning vectors can be used to provide the coding sequences of the therapeutic protein and the polypeptide of the description, such as one nucleic acid molecule containing the coding sequence of the therapeutic protein and the coding sequence of the polypeptide. Expression vectors can be provided to cells in the form of viral vectors. Expression of the polynucleotide of the present description is generally achieved by operationally linking the polynucleotide of the description to a promoter and incorporating the construct into an expression vector. This vector may be suitable for replicating and integrating eukaryotic cells. Typical cloning vectors contain transcription and translation terminators, initiation sequences, and promoters that can be used to regulate the expression of the desired nucleic acid sequence. Viral vector technology is well known in the art and described, for example, in Sambrook et al (2001, Molecular Cloning: A Laboratory Manual, Cold Spring Harbor Laboratory, New York) and other manuals in virology and molecular biology. Viruses that can be used as vectors include, but are not limited to, retroviruses, adenoviruses, adeno-associated viruses, herpesviruses, and lentiviruses. Homologously recombined vectors were used to integrate the expression frame described herein into the host genome.

In general, suitable vectors contain a replication origin, promoter sequence, convenient restriction enzyme sites, and one or more selectable markers functional in at least one organism. For example, in certain embodiments, the description uses a lentiviral vector containing a replication initiation site, a 3' LTR, a 5' LTR, the polynucleotides described herein, and optionally a selectable marker.

An example of a suitable promoter is the immediate early cytomegalovirus (CMV) promoter sequence. This promoter sequence is a strong constitutive promoter sequence capable of driving high-level expression of any polynucleotide sequence operationally linked to it. Another example of a suitable promoter is elongation growth factor-1α (EF-1α). However, other constitutive promoter sequences can also be used, including, but not limited to, simian virus 40 (SV40) early promoter, mouse mammary carcinoma virus (MMTV), human immunodeficiency virus (HIV) long terminal repeat (LTR) promoter, MoMuLV promoter, avian leukaemia virus promoter, Epstein Barr virus immediate early promoter, Rous sarcoma virus promoter, and human gene promoter, such as, but not limited to, actin promoter, myosin promoter, heme promoter, and creatine kinase promoter.

To assess the expression of therapeutic proteins, polypeptides, or portions thereof, the expression vectors introduced into cells may also comprise either or both selectable marker genes or reporter genes to facilitate the identification and selection of the expressing cells from the population of cells transfected or infected by viral vectors. In other respects, selectable markers can be carried over a separate stretch of DNA and used in cotransfection procedures. Both selectable markers and reporter genes can be flanked by appropriate regulatory sequences to enable expression in host cells. Useful selectable markers include, for example, antibiotic resistance genes, such as neo, etc.

Reporter genes were used to identify potentially transfected cells and to evaluate the functionality of regulatory sequences. After DNA has been introduced into recipient cells, the expression of the reporter gene is assayed at an appropriate time. Suitable reporter genes may include genes encoding luciferase, β-galactosidase, chloramphenicol acetyltransferase, secreted alkaline phosphatase, or the green luciferase. Suitable expression systems are well known and can be prepared by known techniques or commercially available.

The polynucleotides described herein can usually be obtained by PCR amplification. In particular, primers can be designed according to the nucleotide sequence disclosed herein, especially the open reading frame sequence, and the sequence of interest can be amplified with a commercially available cDNA library, or a cDNA library prepared by conventional methods known to those skilled in the art as a template. When the sequence is long, two or more PCR amplifications are often required before each of the amplified fragments is spliced together in the correct order. Alternatively, the nucleic acid molecules described herein can also be synthesized directly.

Methods for introducing genes into cells and for expressing genes into cells are known in the art. A vector can be readily introduced into a host cell by any method in the art, e.g., mammalian, bacterial, yeast, or insect cells. For example, an expression vector can be transferred into host cells by physical, chemical, or biological means.

Physical methods for introducing polynucleotides into host cells include calcium phosphate precipitation, lipofection, particle bombardment, microinjection, electroporation, etc. Biological methods for introducing polynucleotides of interest into host cells include the use of DNA and RNA vectors. Chemical means for introducing polynucleotides into host cells include colloidal dispersion systems such as macromolecular complexes, nanocapsules, microspheres, beads; and lipid-based systems, including oil in water emulsions, micelles, mixed micelles, and liposomes.

Biological methods for introducing polynucleotides into host cells comprise the use of viral vectors, particularly retroviral vectors, which have become the most widely used method for inserting genes into mammalian such as human cells. Other viral vectors can be derived from lentiviruses, poxviruses, herpes simplex virus I, adenoviruses, and adeno-associated viruses, etc. Several virus-based systems have been developed for transferring genes into mammalian cells. For example, retroviruses provide a convenient platform for use in gene delivery systems. Techniques known in the art can be exploited to insert selected genes into vectors and package them into retroviral particles. This recombinant virus can subsequently be isolated and delivered to subject cells in vivo or ex vivo. Many retroviral systems are known in the art. Lentiviruses are a genus under the family Retroviridae. Lentiviral vectors are a more complex class of retroviral vectors. Reagents for lentiviral packaging are well known in the art, such as conventional lentiviral vector systems including pRsv-REV, pMDlg-pRRE, pMD2G, and interference plasmids of interest. In one embodiment, the lentiviral vector pWPXL is used.

Thus, in certain embodiments, the description also provides a lentivirus for activating T cells containing the retroviral vector described herein together with the corresponding packaging genes such as gag, pol, vsvg, and/or rev.

In the description, host cells contain, express, and/or secrete the antibodies and/or fusion proteins described herein and, optionally, therapeutic polypeptides. In the description, when it is said that a cell contains or comprises, expresses, secretes a molecule such as a polypeptide, "contain" means that the molecule is included within or on the surface of the cell; "expression" means that the cell produces the molecule; "secretion" means that the cell secretes the expressed molecule out of the cell. Host cells include T cells ultimately used for disease therapeutic purposes, and a variety of cells, such as E. coli cells, which are used in the process of producing CAR-T cells, for example, to provide the coding sequences of the proteins of the description or to provide the vectors described herein. In certain embodiments, a CAR-T cell stably expressing the polypeptide described herein that downregulates the cell surface TCR/CD3 complex is provided.

T cells suitable for the description may be various types of T cells of various origins. For example, T cells can be derived from PBMCs of healthy individuals. In some embodiments, after obtaining T cells, they may be first stimulated and activated with an appropriate amount (e.g., 30-80 ng/ml, such as 50 ng/ml) of CD3 antibody and then cultured in IL2 medium containing an appropriate amount (e.g., 30-80 IU/ml, such as 50 IU/ml) for further use.

Thus, in certain embodiments, the description provides a genetically modified T cell that contains the polynucleotide described herein, or that contains the lentiviral vector described herein, or that has been infected with the lentivirus described herein, or that is prepared using the methods described herein, or that stably expresses the therapeutic proteins and the polypeptides described herein.

T cells of the present description, such as CAR-T cells, can undergo robust in vivo T cell expansion and persist for extended time at high levels in blood and bone marrow, and form specific memory T cells. Without being limited by any particular theory, T cells of the present description can be differentiated in vivo into a central memory like state after encountering and subsequent eliminating target cells expressing the surrogate antigen.

The description also includes a T-cell culture containing the T cells described herein together with a suitable media. Culture media may be those routinely used in the art for T cell culture.

The description also provides a pharmaceutical composition that comprises the T cells described herein as well as pharmaceutically acceptable excipients. In the description, pharmaceutically acceptable excipients refer to vehicles, diluents and/or excipients that are pharmacologically and/or physiologically compatible with the subject and the active ingredient, including but not limited to: pH modifiers, surfactants, carbohydrates, adjuvants, antioxidants, chelators, ionic strength enhancers and preservatives. More specifically, suitable pharmaceutically acceptable excipients may be those commonly used in the art for the administration of T cells, such as CAR-T cells.

Generally, a therapeutically effective amount of T cells is contained in a pharmaceutical composition. A therapeutically effective amount refers to a dose at which the treatment, prevention, mitigation, and/or mitigation of a disease or condition can be achieved in a subject. The effective amount of treatment can be determined by the age, sex, condition and its severity, and other physical condition of a patient. In the description, a subject or patient usually refers to a mammal, especially a human.

A kit containing the nucleic acid constructs described herein is also provided. The kit may also contain various reagents suitable for transfecting said nucleic acid construct into cells, as well as, optionally, instructions directing a person skilled in the art to transfect said recombinant expression vector into cells.

The description also comprises a cell therapy wherein T cells are genetically modified to express the therapeutic proteins and polypeptides described herein and administrated to an object. For example, the administered CAR-T cells are able to kill the recipient's tumor cells. The antitumor immune response caused by CAR-T cells can be an active or passive immune response. Additionally, CAR mediated immune responses may be part of the steps of adoptive immunotherapy, wherein CAR-T cells induce an immune response specific to the antigen binding moiety in CAR.

Accordingly, diseases amenable to treatment with the CARs, polypeptides, their coding sequences, nucleic acid constructs, expression vectors, viruses, or CAR-T cells of the description are related to the tumor antigen single chain antibody contained in the CARs. Accordingly, the diseases described herein encompass various types of cancer associated with tumor antigens as described previously, including solid tumors such as adenocarcinomas, lung cancer, colon cancer, colorectal cancer, breast cancer, ovarian cancer, cervical cancer, gastric cancer, cholangiocarcinomas, gallbladder cancer, esophageal cancer, pancreatic cancer, and prostate cancers, and hematologic neoplasms such as leukemias and lymphomas such as B cell lymphomas, mantle cell lymphomas, acute lymphoblastic leukemias, chronic lymphocytic leukemia, hairy cell leukemia, and acute myeloid leukemia, etc. In embodiments wherein the tumor antigen is CD19, the diseases that can be treated with the CARs comprising the anti-CD19 single chain antibody described herein, polypeptides, their coding sequences, nucleic acid constructs, expression vectors, viruses, or CAR-T cell therapy are preferably CD19 mediated diseases;, for example, acute/chronic B-lineage lymphocytic leukemia, non Hodgkin lymphoma, diffuse large B-cell lymphoma, follicular lymphoma, and mantle cell lymphoma, etc.

The T cells of the present description can be administered alone or as a pharmaceutical composition. The cellular or pharmaceutical compositions of the description may be administered in a manner suitable for the treatment (or prevention) of a disease. The number and frequency of administration will be determined by various factors, such as the patient's condition, and the type and severity of the patient's disease. Administration of the compositions may be carried out in any convenient manner, including by injection, infusion, implantation, or transplantation. The compositions described herein may be administered to the patient subcutaneously, intradermally, intratumorally, intranodally, intraspinally, intramuscularly, by intravenous injection, or intraperitoneally. In one embodiment, the T cell composition of the description is administered to a patient by intradermal or subcutaneous injection. In another embodiment, the T cell composition of the description is preferably administered by intravenous injection. Compositions of T cells can be injected directly into tumors, lymph nodes, or sites of infection.

In some embodiments of the present description, the T cells of the present description or compositions thereof may be combined with other therapies known in the art. The therapies described include, but are not limited to, chemotherapy, radiotherapy and immunosuppressive agents. For example, treatments may be carried out in combination with radiotherapy or chemotherapy formulations that are well known in the art to treat tumor antigen mediated diseases.

In the description, "anti-tumor effect" refers to a biological effect that can be represented by a decrease in tumor volume, a decrease in the number of tumor cells, a decrease in the number of metastases, an increase in life expectancy, or an improvement in various physiological symptoms associated with cancer.

The gene sequence of an anti-CD 19 antibody (specifically, an scFv derived from clone No. FMC63) is used as an example and searched from the NCBI GenBank database for the sequence information of human CD8α hinge region, human CD8 transmembrane region, 4-1BB intracellular region, human CD3ζ intracellular region and the anti-CD3 single chain antibody, a gene fragment of a whole gene synthetic chimeric antigen receptor, which is inserted into a lentiviral vector. The recombinant plasmid packages the virus in 293T cells, infects T cells, and allows the T cells to express the chimeric antigen receptor. The present method of transformation to achieve chimeric antigen receptor gene modified T lymphocytes is based on a lentiviral transformation method. This method has the advantages of high conversion efficiency, stable expression of foreign genes, and can shorten the time for in vitro culture of T lymphocytes to reach a clinical grade number. On the surface of this transgenic T lymphocyte, the transformed nucleic acids are expressed by transcription and translation. CAR-T cells prepared by the present description have strong killing function against specific tumor cells, and the killing efficiency of CAR-T cells containing different anti-CD3 clone single chain antibodies are all over 98% with an effecor-target ratio of 2.5:1.

### Exemplary embodiments

1. An engineered T cell, wherein the T cell expresses a polypeptide that downregulates a cell surface TCR/CD3 complex, the polypeptide comprises a heavy chain variable region (VH) that has an amino acid sequence of SEQ ID No: 1 or more than 95% identical to SEQ ID No: 1, and a light chain variable region (VL) that has an amino acid sequence of SEQ ID No: 2 or more than 95% identical to SEQ ID No: 2.
2. The T cell as described in embodiment 1, wherein
   the VH of the polypeptide that downregulates the cell surface TCR/CD3 complex comprises a sequence of HCDR2: X₁X₂X₃X₄SGYT as shown in SEQ ID No: 9, wherein X₁, X₂, X₃, X₄ are any amino acid, or
   the VH of the polypeptide that downregulates the cell surface TCR/CD3 complex comprises a sequence of HCDR3: ARSX₅X₆X₇DYDGFAY as shown in SEQ ID No: 13, wherein X₅, X₆, X₇ are any amino acid; or
   the VL of the polypeptide that downregulates the cell surface TCR/CD3 complex comprises a sequence of LCDR3: QQWX₈X₉X₁₀X₁₁X₁₂T as shown in SEQ ID No: 15, wherein X₈, X₉, X₁₀, X₁₁, X₁₂ are any amino acid.
3. The T cell as described in embodiment 2, wherein the polypeptide that downregulates the cell surface TCR/CD3 complex comprises has one or more features selected from the group consisting of:
   in the HCDR2 as shown in sequence of SEQ ID No: 9, X₁ amino acid is serine, isoleucine, alanine, valine, leucine, isoleucine, proline, phenylalanine, methionine, tryptophan, threonine, glycine, asparagine, glutamine, tyrosine or cysteine, X₂ amino acid is threonine, asparagine, glycine, glutamine, serine, tyrosine, cysteine, valine or isoleucine, X₃ amino acid is proline, alanine, valine, leucine, isoleucine, phenylalanine, methionine, tryptophan, glycine, asparagine, glutamine, serine, threonine, tyrosine or cysteine, and X₄ amino acid is arginine, lysine, histidine, alanine, valine, leucine, isoleucine, proline, phenylalanine, methionine or tryptophan;
   in the HCDR3 as shown in sequence of SEQ ID No: 13, X₅ amino acid is alanine, glycine, glutamine, serine, threonine, tyrosine, cysteine, lysine, arginine or histidine, X₆ amino acid is tyrosine, alanine, valine, leucine, isoleucine, phenylalanine, methionine, tryptophan, aspartic acid or glutamic acid, X₇ amino acid is tyrosine, lysine, histidine, glycine, asparagine, glutamine, serine, threonine, tyrosine, or cysteine;
   in the LCDR3 as shown in sequence SEQ ID No: 15, X₈ amino acid is serine, alanine, glycine, asparagine, glutamine, threonine, tyrosine, cysteine, alanine, valine, leucine, isoleucine, proline, phenylalanine, methionine, tryptophan, lysine, arginine or histidine, X₉ amino acid is threonine, serine, glycine, asparagine, glutamine, tyrosine, cysteine, lysine, arginine, histidine, phenylalanine, tryptophan, alanine, valine, leucine, isoleucine, proline, methionine, aspartic acid or glutamic acid, X₁₀ amino acid is glutamine, asparagine, glycine, glutamine, serine, threonine, tyrosine, cysteine, valine, isoleucine, alanine, leucine, proline, phenylalanine, methionine, tryptophan, lysine, arginine or histidine, X₁₁ amino acid is proline, alanine, valine, leucine, isoleucine, phenylalanine, methionine, tryptophan, threonine, isoleucine, lysine, arginine, histidine, tyrosine, tryptophan, glycine, asparagine, glutamine, serine or cysteine, X₁₂ amino acid is glycine, asparagine, glutamine, serine, threonine, tyrosine, cysteine, alanine, valine, leucine, isoleucine, proline, phenylalanine, methionine, tryptophan.
4. The T cell as described in any of embodiments 1-3, wherein the polypeptide that downregulates the cell surface TCR/CD3 complex comprises one or more of each of the following groups:
   (1) One of the following LCDR3 sequences: SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID NO: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34,
   (2) One of the following HCDR2 sequences: SEQ ID NO: 10, SEQ ID NO: 11, SEQ ID NO: 12,
   (3) HCDR3 sequence: SEQ ID NO: 14.
5. The T cell as described in any of embodiments 1-4, wherein the polypeptide is a single chain antibody.
6. The T cell as described in any of embodiments 1-5, wherein the polypeptide that downregulates the cell surface TCR/CD3 complex comprises a localization sequence and optionally a signal peptide, preferably,
   the localization sequence is selected from the group consisting of endoplasmic reticulum retention sequence, Golgi retention sequence, E3 ubiquitin ligase binding sequence, proteasome localization sequence, or lysosome localization sequence; preferably, the endoplasmic reticulum retention sequence comprises or consists of any of the sequences shown in SEQ ID Nos: 35-40, where X is any amino acid,
   a linker is included between the localization sequence and the antibody, preferably as shown in SEQ ID No: 41;
   the signal peptide is shown in positions 516-537 of SEQ ID No: 44.
7. The T cell as described in any of embodiments 1-6 further expresses a therapeutic protein, preferably the therapeutic protein is a chimeric antigen receptor.
8. The T cell as described in embodiment 7, wherein the T cell contains an expression frame of the therapeutic protein and an expression frame of the polypeptide that downregulates the cell surface TCR/CD3 complex, or a coding sequence of the therapeutic protein and a coding sequence of the polypeptide that downregulates the cell surface TCR/CD3 complex are in the same expression frame.
9. An engineered T cell, wherein said T cell expresses a polypeptide that downregulates a cell surface TCR/CD3 complex, the polypeptide comprises: an antibody or an antigen binding fragment thereof, or a mutant having at least 90% sequence identity with the antibody or the antigen binding fragment thereof and retaining antigen binding activity of the antibody or the antigen binding fragment thereof; wherein, the antibody comprises a heavy chain variable region and a light chain variable region,
   the heavy chain variable region has the following HCDRs:
   HCDR1 as shown in SEQ ID NO:3: GYTFISYT,
   HCDR2 as shown in SEQ ID NO:9: X₁X₂X₃X₄SGYT, wherein X₁, X₂, X₃, X₄ are any amino acid,
   HCDR3 as shown in SEQ ID NO:13: ARSX₅X₆X₇DYDGFAY, wherein X₅, X₆, X₇ are any amino acid,
   the light chain variable region has the following LCDRs:
   LCDR1 as shown in SEQ ID NO:6: SSVSY,
   LCDR2 as shown in SEQ ID NO:7: DTS,
   LCDR3 as shown in SEQ ID NO:15: QQWX₈X₉X₁₀X₁₁X₁₂T, wherein X₈, X₉, X₁₀, X₁₁, X₁₂ are any amino acid.
10. The T cell as described in embodiment 9, wherein the polypeptide that downregulates the cell surface TCR/CD3 complex has one or more features selected from the group consisting of:
   X₁ is serine, isoleucine, alanine, valine, leucine, isoleucine, proline, phenylalanine, methionine, tryptophan, threonine, glycine, asparagine, glutamine, tyrosine, or cysteine,
   X₂ is threonine, asparagine, glycine, glutamine, serine, tyrosine, cysteine, valine, or isoleucine,
   X₃ is proline, alanine, valine, leucine, isoleucine, phenylalanine, methionine, tryptophan, glycine, asparagine, glutamine, serine, threonine, tyrosine, or cysteine,
   X₄ is arginine, lysine, histidine, alanine, valine, leucine, isoleucine, proline, phenylalanine, methionine, or tryptophan,
   X₅ is alanine, glycine, glutamine, serine, threonine, tyrosine, cysteine, lysine, arginine, or histidine,
   X₆ is tyrosine, alanine, valine, leucine, isoleucine, phenylalanine, methionine, tryptophan, aspartate, or glutamate,
   X₇ is tyrosine, lysine, histidine, glycine, asparagine, glutamine, serine, threonine, tyrosine, or cysteine,
   X₈ is serine, alanine, glycine, asparagine, glutamine, threonine, tyrosine, cysteine, alanine, valine, leucine, isoleucine, proline, phenylalanine, methionine, tryptophan, lysine, arginine, or histidine,
   X₉ is threonine, serine, glycine, asparagine, glutamine, tyrosine, cysteine, lysine, arginine, histidine, phenylalanine, tryptophan, alanine, valine, leucine, isoleucine, proline, methionine, aspartic acid, or glutamic acid,
   X₁₀ is glutamine, asparagine, glycine, glutamine, serine, threonine, tyrosine, cysteine, valine, isoleucine, alanine, leucine, proline, phenylalanine, methionine, tryptophan, lysine, arginine, or histidine,
   X₁₁ is proline, alanine, valine, leucine, isoleucine, phenylalanine, methionine, tryptophan, threonine, isoleucine, lysine, arginine, histidine, tyrosine, tryptophan, glycine, asparagine, glutamine, serine, or cysteine,
   X₁₂ is glycine, asparagine, glutamine, serine, threonine, tyrosine, cysteine, alanine, valine, leucine, isoleucine, proline, phenylalanine, methionine, tryptophan.
11. The T cell as described in embodiments 9 or 10, wherein the polypeptide that downregulates the cell surface TCR/CD3 complex has one or more features selected from the group consisting of:
   LCDR3 is selected from the group consisting of: SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34,
   HCDR2 is selected from the group consisting of: SEQ ID NO:10, SEQ ID NO:11, SEQ ID NO:12,
   HCDR3 is SEQ ID NO:14.
12. The T cell as described in any of embodiments 9-11, wherein,
   the amino acid sequence of the heavy chain variable region is selected from:
      (1) SEQ ID NO:42,
      (2) a variant of SEQ ID No: 42 containing a sequence of HCDR2 and/or HCDR3 as described in embodiments 10 or 11
      (3) sequences with more than 95% identity to (1) or (2), and/or
   the amino acid sequence of the light chain variable region is selected from:
      (1) SEQ ID NO:43,
      (2) a variant of SEQ ID No: 43 containing a sequence of LCDR3 as described in embodiments 10 or 11;
      (3) a sequence with more than 95% identity to (1) or (2).
13. The T cell as described in any of embodiments 9-12, wherein the antibody is a single chain antibody.
14. The T cell as described in any of embodiments 9-12, wherein the polypeptide that downregulates the cell surface TCR/CD3 complex comprises a localization sequence and optionally a signal peptide, preferably,
   the localization sequence is selected from the group consisting of: endoplasmic reticulum retention sequence, Golgi retention sequence, E3 ubiquitin ligase binding sequence, proteasome localization sequence, or lysosome localization sequence; preferably, the endoplasmic reticulum retention sequence comprises or consists of any of the sequences shown in SEQ ID Nos: 35-40, where X is any amino acid;
   a linker is included between the localization sequence and the antibody, preferably as shown by SEQ ID No: 41,
   the signal peptide is shown in positions 516-537 of SEQ ID No: 44.
15. The T cell as described in any of embodiments 9-14, further expresses a therapeutic protein; preferably, the therapeutic protein is a chimeric antigen receptor.
16. The T cell as described in embodiment 15, wherein the T cell contains an expression frame of the therapeutic protein and an expression frame of the polypeptide that downregulates the cell surface TCR/CD3 complex, or a coding sequence of the therapeutic protein and a coding sequence of the polypeptide that downregulates the cell surface TCR/CD3 complex are in the same expression frame.
17. An antibody or an antigen binding fragment thereof, wherein the antibody has an amino acid sequence of HCDR1 of GYTFISYT, an amino acid sequence of HCDR2 of X₁X₂X₃X₄SGYT, an amino acid sequence of HCDR3 of ARSX₅X₆X₇DYDGFAY, an amino acid sequence of LCDR1 of SSVSY, an amino acid sequence of LCDR2 of DTS, an amino acid sequence of LCDR3 of QQWX₈X₉X₁₀X₁₁X₁₂T; wherein, X₁-X₁₂ are as described in embodiment 10, and HCDR2 is not INPRSGYT, HCDR3 is not ARSAYYDYDGFAY, LCDR3 is not QQWSSNPPT,
   preferably, the LCDR3, HCDR2 and HCDR3 of the antibody are as described in embodiment 11.
18. The antibody or the antigen binding fragment thereof as described in embodiment 17, wherein
   the amino acid sequence of the heavy chain variable region of the antibody is shown in SEQ ID No: 42, but the sequences of its HCDR2 and/or HCDR3 are as described in embodiments 10 or 11;
   the amino acid sequence of the light chain variable region of the antibody is shown in SEQ ID No: 43, but the sequence of its LCDR3 is as described in embodiments 10 or 11.
19. A fusion protein comprising a localization sequence, a polypeptide that downregulates a cell surface TCR/CD3 complex, and, optionally, a signal peptide; preferably, the polypeptide that downregulates the cell surface TCR/CD3 complex is as described in any of embodiments 9-18, preferably,
   the signal peptide is shown in positions 516-537 of SEQ ID No: 44,
   a linker is included between the localization sequence and the polypeptide that downregulates the cell surface TCR/CD3 complex, preferably as shown by SEQ ID No: 41,
   the fusion proteins are as shown in positions 516-804 of SEQ ID No: 44 or as shown in positions 538-804 of SEQ ID No: 44.
20. A nucleic acid molecule, wherein the nucleic acid molecule is selected from the group consisting of:
   (1) a nucleic acid molecule comprising a coding sequence of a therapeutic protein and a coding sequence of a polypeptide that downregulates a cell surface TCR/CD3 complex;
   (2) the coding sequence of the fusion protein as described in embodiment 19;
   (3) the coding sequence of the antibody or the antigen binding fragment thereof of embodiments 17 or 18; and
   (4) a complementary sequence or fragment of (1), (2), or (3);
   Preferably, the therapeutic protein is a chimeric antigen receptor, and the polypeptide that downregulates the cell surface TCR/CD3 complex is as described in embodiments 9-14.
21. A nucleic acid construct, wherein the nucleic acid construct comprises a nucleic acid molecule as described in embodiment 20,
   preferably, the nucleic acid construct contains an expression frame of the therapeutic protein and an expression frame of the polypeptide that downregulates the cell surface TCR/CD3 complex; or the nucleic acid construct is one expression frame wherein a coding sequence of the therapeutic protein and a coding sequence of the polypeptide that downregulates the cell surface TCR/CD3 complex are in this expression frame; or
   the nucleic acid construct is a cloning vector or expression vector.
22. A lentivirus wherein the lentivirus contains a nucleic acid molecule described in embodiment 20 or a nucleic acid construct described in embodiment 21.
23. A host cell, wherein the host cell comprises, expresses and/or secrets an antibody or an antigen binding fragment thereof as described in embodiments 17 or 18, or a fusion protein as described in embodiment 19, and optionally a therapeutic protein; preferably, the host cell comprises a nucleic acid molecule as described in embodiment 20, a nucleic acid construct as described in embodiment 21 and/or a lentivirus as described in embodiment 22.
24. A pharmaceutical composition comprising a T cell as described in any of embodiments 9-16, an antibody or an antigen binding fragment thereof as described in embodiments 17 or 18, a fusion protein as described in embodiment 19, a nucleic acid molecule as described in embodiment 20, a nucleic acid construct as described in embodiment 21 and/or a lentiviruse as described in embodiment 22, and a pharmaceutically acceptable carrier.
25. Use of a polypeptide that downregulates a cell surface TCR/CD3 complex or its coding sequence, a nucleic acid molecule as described in embodiment 20, a nucleic acid construct as described in embodiment 21 and/or a lentivirus as described in embodiment 22 in the preparation of a T cell with downregulated cell surface TCR, or in the preparation of T cells for cancer therapy, wherein the polypeptide that downregulates a cell surface TCR/CD3 complex is as described in embodiments 9-14.

The description is further described in detail by reference to the following experimental examples. These examples are provided for illustrative purposes only and are not intended to be limiting unless otherwise specified. Thus, the present description should by no means be understood as being limited to the following examples, but rather should be understood as including any and all of the variations that become apparent as a result of the teaching provided herein. The methods and reagents used in the examples, unless otherwise stated, are those conventional in the art.

### EXAMPLES

### Example 1. Construction of vector

1. The gene sequences of human CD8α hinge region, human CD8 transmembrane region, 4-1BB intracellular region, human CD3ζ intracellular region, heavy and light chain variable regions of an anti-human CD19 antibody (clone FMC63), and heavy and light chain variable regions of a control anti-human CD3 antibody (clones UCHT1 and OKT3) were searched from the NCBI website database. The remaining antibody sequences were derived from specific anti-human CD3 antibody clones. All relevant amino acid sequences were subject to codon optimization at the website https://www.thermofisher.com/order/geneartgenes, which made them more suitable for expression by human cells without a change in the encoded amino acid sequence.
2. Overlapping PCR was used to link above sequences in turn as follows: anti-CD 19-scFv gene, human CD8 hinge region gene, human CD8 transmembrane region gene, 4-1BB intracellular region gene, human CD3ζintracellular region, F2A and CD3-scFv gene, and endoplasmic reticulum retention sequence (AEKDEL) to form complete gene sequence information. Its sequence comprising a coding sequence of a signal peptide was shown as residues 1-66 or 1546-1611 of SEQ ID No: 51, and its amino acid sequence was shown as residues 1-22 and 516-537 of SEQ ID No: 44.
3. The nucleotide sequence of the CAR molecule was seamlessly cloned into the BamHI/EcoRI sites of the lentiviral plasmid pWPXL (Addgene) and transformed into competent *E. coli* (Stbl3).
4. The recombinant plasmids were sent to Suzhou Genewiz Bio-Technology Co., Ltd. for sequencing, and the sequencing results were aligned with the sequence to be synthesized to verify that the sequence was correct. The sequencing primers were:
   Forward sequence: TCAAGCCTCAGACAGTGGTTC (SEQ ID NO:49)
   Reverse sequence: CCAGTCAATCTTTCACAAATTTTG (SEQ ID NO:50).

### Example 2. Viral packaging

After sequencing to be correct, the plasmids were extracted and purified using a plasmid purification kit from Qiagen, and the purified plasmids were transfected into 293T cells by the calcium phosphate method for lentiviral packaging experiments (Molecular Therapy-Methods & Clinical Development, 2016, 3: 16017), the obtained lentiviral vectors were as follows: Control (CAR19-F2A-GFP), Sample 1 (CAR19-F2A-UCHT1 scfv-AEKDEL), Sample 2 (CAR19-F2A-OKT3 scfv-AEKDEL), Samples 3 (CAR19-F2A-CTAB1 scfv-AEKDEL), Sample 4 (CAR19-F2A-CTAB2 scfv-AEKDEL), Sample 5 (CAR19-F2A-CTAB3 scfv-AEKDEL), Sample 6 (CAR19-F2A-CTAB4 scfv-AEKDEL), and Sample 7 (CTAB1 scfv-AEKDEL).

### Example 3. Isolation of PBMCs and T cells

Isolation of PBMCs from peripheral blood, isolation and activation of T cells, lentiviral transduction, ex vivo culture

Healthy donors who tested negative for HBV, HCV, and HIV were selected, blood was drawn from the median cubital vein, the buffer coat of PBMCs was separated by Ficoll density gradient centrifugation, the percentage of CD3+ T cells was determined according to flow cytometry of whole blood, the number of CD3+ T cells was calculated, and a use amount of magnetic beads were taken according to the ratio of Dynabeads CD3/CD28 to CD3+ T cells of 3:1, incubated with the buffer coat cells for 30 min, CD3+ T cells were isolated, then activated with Dynabeads CD3/CD28 (Life Technology) for 24 hours, and the proportion of CD25+CD69+ T cells was determined by flow cytometry.

### Example 4. Lentiviral transduction and T cell culture

After CD3+ T activation, lentiviral transduction was performed. The 24 well plates coated with Novonectin were incubated at 37°C for 2 hours, and the cell suspension was formulated with various lentiviruses prepared as described previously (MOI = 3), Tscm (2U/ml, Novoprotein) into transduction systems, respectively, which were placed in coated 24 well plates, and the cell density was adjusted to 1.0E+06/ml and centrifuged at 500 g for 30 minutes, followed by incubation in an incubator at 37°C and 5% CO₂ for 48 hours. After lentiviral transfection, the cells were cultured in Xvivo15 medium with 5% FBS, supplemented with Tscm (to final concentration of 2U/ml) every other day, counted, and adjusted to 0.5E+06/ml, and harvested after culture until Day 8-10.

### Example 5. CAR positive rate and CAR-T cell TCR or CD3 mean fluorescence intensity

5.0E+05 cells were counted and collected, and the cells were washed 2 times and resuspended in 100 UL of buffer containing 4% BSA. 8 ul of anti-human TCR or CD3 antibody was added to each tube of cells, vortexed to mix, and incubated at 4°C for 30 minutes. After staining and incubation, the cells were washed repeatedly, and fluorescently labeled anti-CAR19 antibody or Protein L was diluted 500×, and cells were resuspended in 200 ul per tube by vortexing and incubated at 4°C for 30 minutes. The cells were washed repeatedly and resuspended in 500ul of buffer containing 4% BSA, 4ul of 7AAD dye was added to each tube, vortexed to mix, and incubated at room temperature for 10 min in dark. Finally, the samples were transferred to flow tubes, and the efficiency of CAR19 transfection as well as the surface TCR or CD3 levels of CAR19+ T cell populations were detected on Calibr flow cytometer.

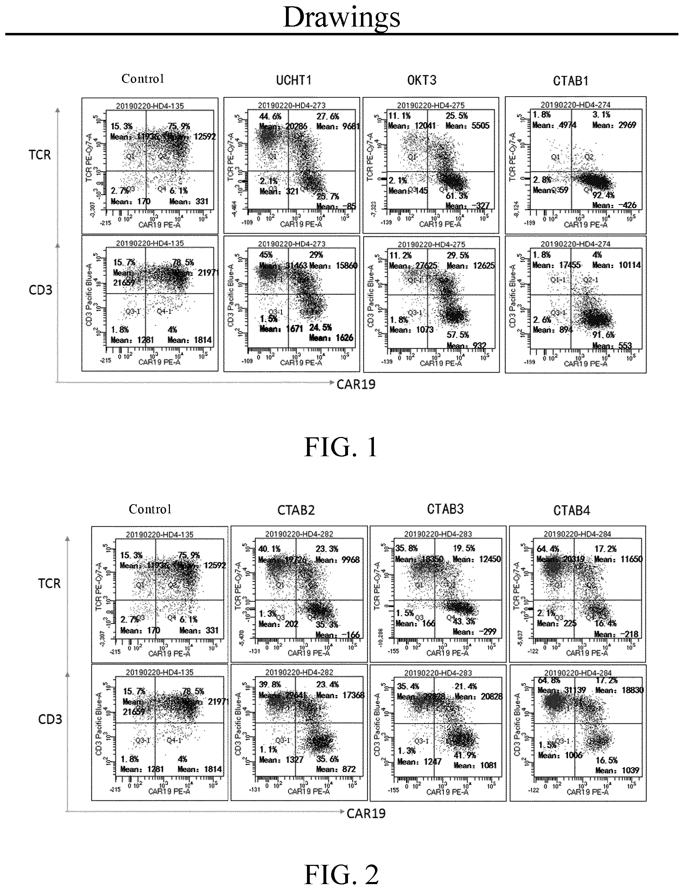

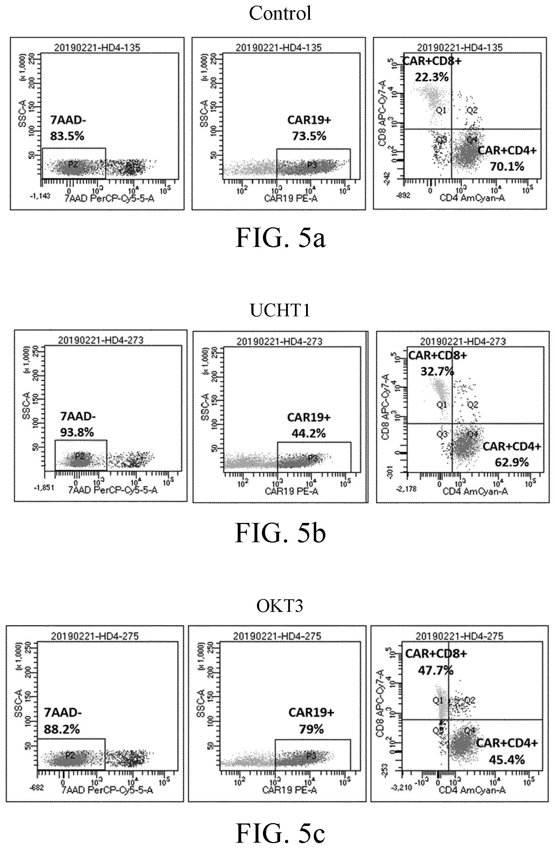

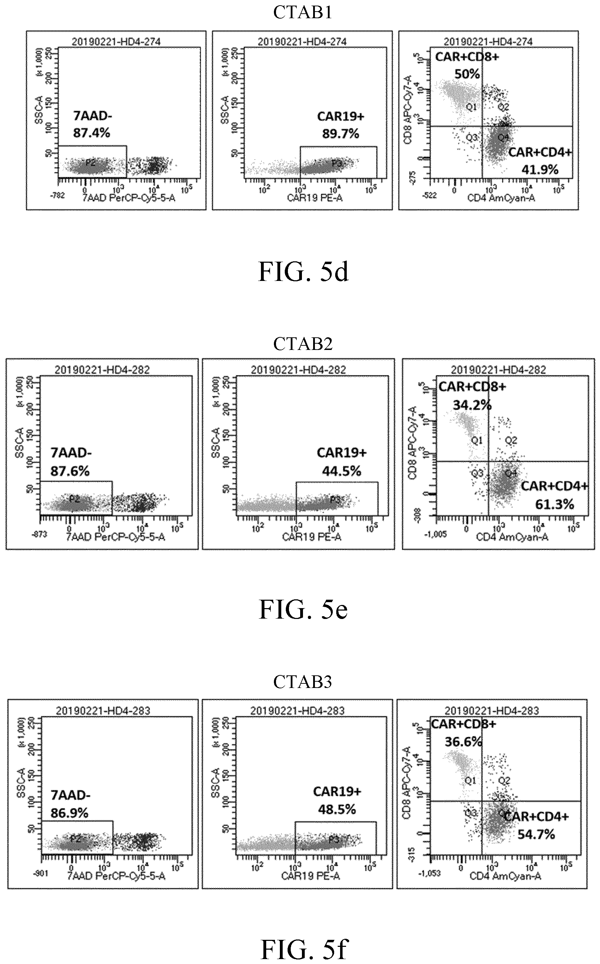

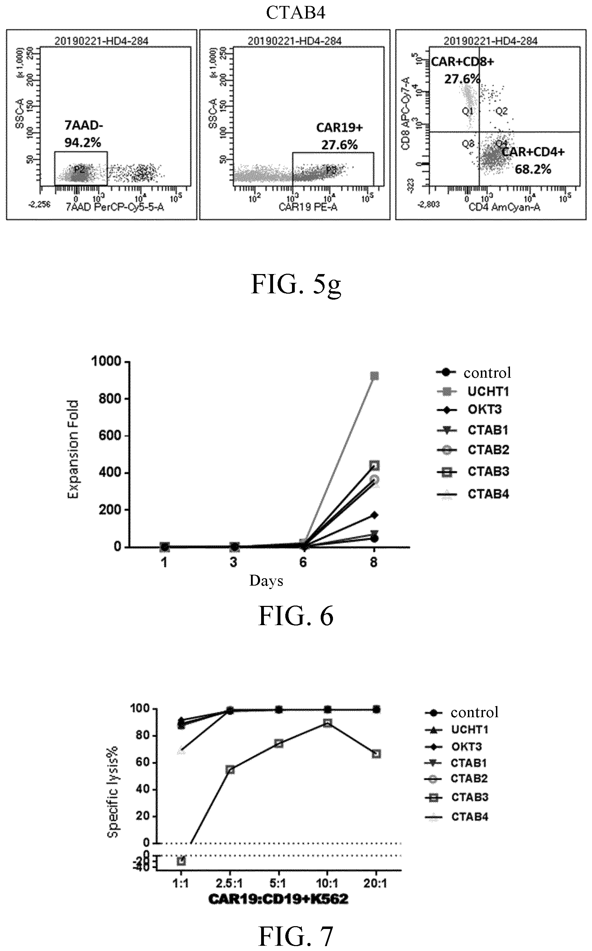

Two widely used murine derived anti-human CD3 antibodies (OKT3 and UCTH1) were first evaluated. To avoid rejection by murine derived sequences in clinical applications, they were humanized and tested at the cellular level. The results were shown in FIG. 1 and Table 1, expression of a peptide containing the sequence of the humanized UCHT1 antibody showed some activity in downregulating the cell surface TCR/CD3 complex, and in transduced positive cells (CAR+), the percentages of the TCR- and CD3- populations were 48.22% and 45.8%, respectively. US Patent US20180086831A1 also tested UCHT1 antibody sequences, and its FIG. 5 showed that the levels of surface downregulation of the TCR/CD3 complex varied largely for T cells from different donors. For donor 1, the proportion of the TCR- population in transfection positive cells was 17.1/(17.1 + 14.1)^{∗} 100% = 54.8%, which was close to the results obtained in the present description. However, for donor 2, the proportion of the TCR- population in transfection positive cells was 42.5/(42.5 + 6.79)^{∗} 100% = 86.2%, exhibiting a large difference from donor 1.

The peptide containing the humanized OKT3 sequence downregulated the activity of the cell surface TCR/CD3 complex more than those containing the UCH1 sequence (FIG. 1 and Table 1). In CAR+ cells, the percentages of TCR- and CD3- populations were elevated to 70.62% and 66.09%, respectively, but the percentages of TCR/CD3+ population were still higher, which would bring great challenges to the purification of CAR-T cells and also great risks to clinical applications. The peptides containing the sequences of the other three anti-CD3 antibodies (FIG. 2 and Table 1) were close in activity to the peptides containing OKT3 sequence, and the proportion of CAR⁺TCR⁻/CAR⁺ ranged from 48.81 to 68.95%.

Unexpectedly, a peptide containing the CTAB1 sequence of the antibody was able to most efficiently downregulate the level of the cell surface TCR/CD3 complex, with up to 96.75% of CAR+ cells expressing no surface TCR; up to 95.82% of CAR+ cells expressing no surface CD3 (FIG. 1 and Table 1).

**Table 1**

| | Control | UCHT1 | OKT3 | CTAB1 | CTAB2 | CTAB3 | CTAB4 |
|---|---|---|---|---|---|---|---|
| The proportion of the TCR negative cell population in the transduced positive cells^{a} | 7.44% | 48.22% | 70.62% | 96.75% | 60.24% | 68.95% | 48.81% |
| The percentage of the CD3 negative cell population in the transduced positive cells^{a} | 4.85% | 45.80% | 66.09% | 95.82% | 60.34% | 66.20% | 48.96% |

| | | | | | | | |
|---|---|---|---|---|---|---|---|
| a: the proportion of Q4/the proportion of Q2+Q4 | | | | | | | |

### Example 6. CAR positive rates of T cells from different donor as well as surface TCR or CD3 protein levels of CAR-T cells

The CAR-T cells from different donors containing CTAB1 sequence polypeptides were prepared according to the procedure of Examples 3 and 4, and the efficiency of CAR19 transfection as well as the TCR or CD3 mean fluorescence intensity of the CAR19+ T cell populations were determined by flow cytometry according to Example 5.

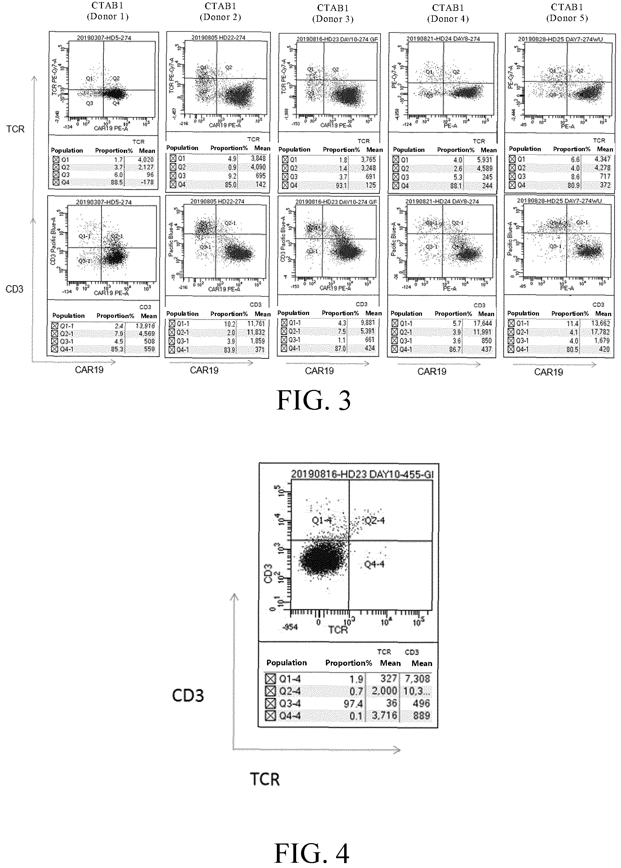

Results were as shown in FIG. 3, peptides expressing CTAB1 sequence exhibited the ability to stably downregulate the cell surface TCR/CD3 complex in CAR-T cells from different donors. Calculated results were as shown in Table 2, the proportion of the TCR negative population in the transfection positive cells (CAR+) was no less than 95% among the different donors, showing that peptides containing CTAB1 sequence were applicable to T cells from a wide range of donors.

**Table 2**

| | CTAB1 (donor 1) | CTAB1 (donor 2) | CTAB1 (donor 3) | CTAB1 (donor 4) | CTAB1 (donor 5) |
|---|---|---|---|---|---|
| The proportion of the TCR negative cell population in the transduced positive cells^{a} | 95.99% | 98.95% | 98.52% | 97.13% | 95.29% |
| The proportion of the CD3 negative cell population in the transduced positive cells^{a} | 91.52% | 97.67% | 92.06% | 95.70% | 95.15% |

| | | | | | |
|---|---|---|---|---|---|
| a: the proportion of Q4/the proportion of Q2+Q4 | | | | | |

### Example 7. Expression of a peptide containing CTAB1 antibody sequence efficiently downregulates surface TCR and CD3 levels of T cells

To confirm the activity of the polypeptide containing CTAB1 antibody sequence, lentiviral vectors expressing only the polypeptide containing CTAB1 sequence but not the therapeutic protein were prepared as in Example 2, the corresponding T cells were prepared as in Examples 3 and 4, and the TCR or CD3 levels on their surfaces were detected by flow cytometry as in Example 5.

Results were as shown in FIG. 4, the polypeptides containing CTAB1 antibody sequence, when expressed alone, were still able to efficiently inhibit the expression of surface TCR and CD3.

### Example 8. Cellular phenotyping

The CAR-T cells in each group were counted, and the 1.0E+06 cells were collected by centrifugation, and the cells were washed twice by resuspension with sterile 4% BSA, followed by resuspension with 200ul 4% BSA. Anti-human antibodies as follows were added to each sample: CD4 (AmCyan), CD8 (APC-Cy7), CD45RA (PE-Cy7) and CCR7 (BV421). 5 ul of each antibody was added, mixed well by vortex homogenizer and incubated at 4°C for 30 minutes. After staining and incubation, the cells were washed repeatedly and resuspended with 500ul of 4% BSA buffer, 4ul of 7AAD dye was added to each tube, vortexed and incubated at room temperature in dark for 10 minutes. Samples were transferred to flow tubes, and T cell immunophenotypes were analyzed for CCR7 and CD45RA expression on T cells from each group on FACS Calibr flow cytometer.

The results were shown in FIGs. 5a-5g. The proportions of CAR+CD8+ T and CAR+CD4+ T in each group were normal and not significantly different between groups, including the group of cells with the most potent polypeptide containing CTAB1 antibody sequence that downregulated the cell surface TCR/CD3 complex.

### Example 9. T cell in vitro expansion assay

Each group of T cells transfected with lentiviral vector, T cells were counted every other day, T cells were cultured in vitro to Day 8, and the cell proliferation fold (cell number on Day 8/cell number used for transfection) was calculated.

The results were shown in FIG. 6. The expansion capacity of T cells with downregulated surface expression of TCR/CD3 complex in each group was not attenuated but improved to various degrees compared with control.

### Example 10. T cell killing of tumour target cells

NC-T cells (T cells without lentiviral transfection) and CAR-T cells in each group were counted and collected, and the cell density was adjusted to 5.0E+07/ml with T cell medium X-VIVO15 (without Tscm). The target cells were counted and collected, and the cell density was adjusted to 5.0E+06/ml with RPMI 1640 (without FBS). The above adjusted densities of effector cells were mixed with target cells at different effector to target ratios (1:1, 2.5:1, 5:1, 10:1, 20:1) in a 1.5 ml centrifuge tube, and the total volume was made up to 200ul with X-VIVO15. They were moved into 96 well V-bottom plate and incubated at 37°C and 5% CO₂ for 24 h, gently pipetted to mix the cells of each well and 100ul of cell suspension was transferred into a 96 well plate with white wall solid bottom. 80 µl ONE-Glo^{™} Luciferase substrate was added, mixed by pipetting, and detected for the fluorescence intensity on Luminoskan Ascent chemiluminescence analyzer after incubation in dark at room temperature for 10 minutes.

The results were shown in Table 3 and FIG. 7. Except for cells expressing peptides containing CTAB3 antibody sequence, all other groups showed ability of efficiently killing tumor target cells.

**Table 3**

| name | 1:1 | 2.5:1 | 5:1 | 10:1 | 20:1 |
|---|---|---|---|---|---|
| control | 89.15% | 98.36% | 99.27% | 99.52% | 99.46% |
| UCHT1 | 87.82% | 99.12% | 99.72% | 99.61% | 99.94% |
| OKT3 | 91.74% | 98.51% | 99.51% | 99.50% | 99.72% |
| CTAB1 | 87.85% | 98.88% | 99.50% | 99.58% | 99.98% |
| CTAB2 | 87.81% | 99.16% | 99.61% | 99.64% | 99.84% |
| CTAB3 | -19.07% | 55.03% | 74.29% | 89.55% | 66.60% |
| CTAB4 | 69.40% | 99.17% | 99.68% | 99.82% | 99.65% |

### Example 11. The ability of IFN-γ secretion by T cells

Lentivirus transfected T cells from each group were cultured in vitro until Day 8, and the TCR dependent T cell activating antibody OKT3 was added to final concentrations as follows: 50 ng/ml, 100 ng/ml, 150 ng/ml, and 200 ng/ml. Each group of CAR-T cell samples was diluted by appropriate fold with X-VIVO15 media, corresponding number of strips were removed as required, 100 ul of dilution RD1-51 was added to each well, diluted samples were mixed and 100 ul was taken into wells (completed within 15 min); covered with a parafilm and incubated for 2 hours at room temperature. After the incubation was complete, the solution in the wells was completely removed, wash solution was added, standed for 1 min, this step was repeated for a total of four washes; 200 ul of human IFN-γ coupling reagent was added to each well, covered with a new parafilm and incubated for 2 hours at room temperature; 200 ul of substrate solution was added to each well and incubated for 30 min in dark at room temperature; after the incubation was completed, 50 ul of stop solution was added to each well, mixed well and then detected on a microplate reader (wavelength set at 450 nm, corrected at 570 nm/540 nm).

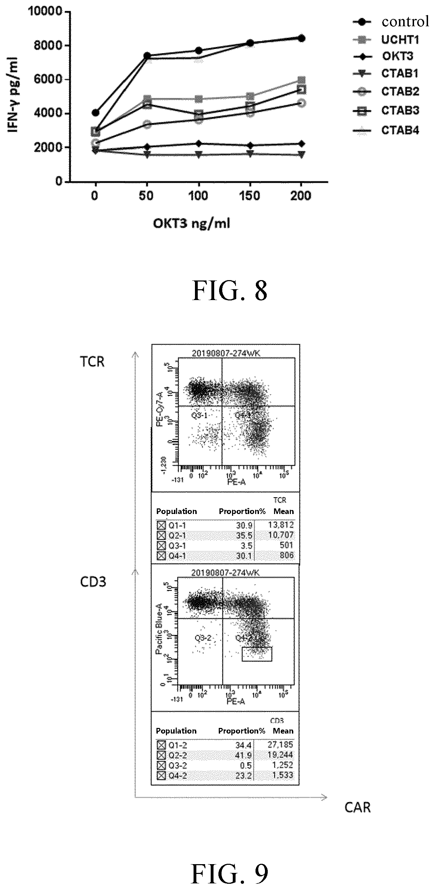

The results were shown in Table 4 and FIG. 8. The secretion of IFN-γ was downregulated in all experimental groups compared with control, wherein the ability of IFN-γ secretion in cells containing CTAB1 antibody sequence was completely abolished, indicating the absence of a functional TCR/CD3 complex on the cell surface of these cells, which was fully consistent with the results of the aforementioned experiments.

**Table 4**

| Sample | OKT3 (ng/ml) | OD value | INF-γ (pg/ml) | Sample | OKT3 | OD value | INF-γ (pg/ml) |
|---|---|---|---|---|---|---|---|
| | | 10 fold dilution | | | | 10 fold dilution | |
| control | 0 | 0.992 | 4063.9 | CTAB2 | 0 | 0.56 | 2270.9 |
| | 50 | 1.801 | 7414.7 | | 50 | 0.824 | 3366.7 |
| | 100 | 1.875 | 7718.8 | | 100 | 0.892 | 3649.2 |
| | 150 | 1.982 | 8165.4 | | | | |
| | | | | | 150 | 0.99 | 4054.0 |
| | 200 | 2.048 | 8439.2 | | 200 | 1.129 | 4630.6 |
| UCHT1 | 0 | 0.702 | 2860.0 | CTAB3 | 0 | 0.725 | 2957.3 |
| | 50 | 1.187 | 4871.8 | | 50 | 1.108 | 4544.9 |
| | 100 | 1.186 | 4864.3 | | 100 | 0.968 | 3963.2 |
| | 150 | 1.224 | 5022.2 | | 150 | 1.084 | 4443.4 |
| | 200 | 1.455 | 5981.6 | | | | |
| | | | | | 200 | 1.323 | 5433.1 |
| OKT3 | 0 | 0.458 | 1852.0 | CTAB4 | 0 | 0.741 | 3021.6 |
| | 50 | 0.507 | 2053.8 | | 50 | 1.759 | 7238.6 |
| | 100 | 0.553 | 2242.3 | | 100 | 1.769 | 7280.4 |
| | 150 | 0.527 | 2136.6 | | 150 | 1.976 | 8140.1 |
| | 200 | 0.552 | 2237.7 | | 200 | 2.069 | 8522.5 |
| CTAB1 | 0 | 0.459 | 1853.7 | | | | |
| | 50 | 0.396 | 1593.1 | | | | |
| | 100 | 0.397 | 1596.8 | | | | |
| | 150 | 0.411 | 1655.2 | | | | |
| | 200 | 0.397 | 1595.2 | | | | |

### Example 12. Preparation and screening of a mutant library

1) The target CD3 antibody sequence was selected for amino acid point mutations to search for mutants that more effectively downregulated surface TCR/CD3 complex expression, or eliminated its MHC-I epitope, or could promote application advantages such as T cell expansion. Position 1 of the mutant library was located in the light chain of the target scFv (amino acids 90-95 of SEQ ID No: 43, WSSNPP), position 2 was located in the heavy chain of the target scFv (amino acids 50-54 of SEQ ID No: 42, YINPR), and position 3 was located in the heavy chain of the target scFv (amino acids 98-102 of SEQ ID No: 42, RSAYY).
2) NNK degenerate primers were designed to carry out the construction of above mutation library. The resulting library of lentiviral target mutants was prepared following the procedure of Examples 1 and 2.
3) The resulting mutant T cell library of interest was prepared following the procedure of Examples 3 and 4.
4) The TCR and CD3 expression profile of the target mutant T cell library was examined as in Example 5. Results were as shown in FIG. 9, the target mutation library cells exhibited the ability of downregulating the expression of TCR and CD3.
5) The subset of T cell clones with the highest capacity to downregulate TCRs was flow sorted, and genomic high-throughput sequencing was performed to determine the results of final mutation library, which were shown in Table 5, demonstrating the top 23 mutants with the highest proportion in the library. The discovered mutants could be further used to analyze whether they conferred any application advantage, including, but not limited to, increasing the efficiency of downregulating the cell surface TCR/CD3 complex, accelerating the in vitro expansion of T cells, or eliminating MHC epitope alleles present in the primary sequence.
6) Exemplarily, based on the library results in Table 5, the found mutant sequences were analyzed at the website http://www.iedb.org/home v3.php for MHC-I epitope, according to the website analysis results, it showed that mutant VL-CDR3: QQWSTQRPT (SEQ ID No: 25) and mutant VL-CDR3: QQWSGQPPT (SEQ ID No: 27) abolished MHC-I epitope in the target antibody sequence of CD3 antibody.

**Table 5**

| Frequency of sequencing results | Position of mutation | Amino acid sequence | SEQ ID NO. |
|---|---|---|---|
| 2.80% | VL-CDR3 | QQWSSTVPT | SEQ ID NO:16 |
| 2.59% | VL-CDR3 | QQWAHNPPT | SEQ ID NO:17 |
| 1.89% | VL-CDR3 | QQWSYVRPT | SEQ ID NO:18 |
| 1.60% | VL-CDR3 | QQWSFVSPT | SEQ ID NO:19 |
| 1.41% | VH-CDR2 | ITPRSGYT | SEQ ID NO:10 |
| 1.36% | VH-CDR2 | ITTLSGYT | SEQ ID NO:11 |
| 1.35% | VH-CDR3 | ARSRESDYDGFAY | SEQ ID NO:14 |
| 1.13% | VL-CDR3 | QQWAMSPPT | SEQ ID NO:20 |
| 1.24% | VL-CDR3 | QQWSSWPPT | SEQ ID NO:21 |
| 1.14% | VH-CDR2 | SNPRSGYT | SEQ ID NO:12 |
| 1.08% | VL-CDR3 | QQWSQPHTT | SEQ ID NO:22 |
| 1.08% | VL-CDR3 | QQWSNQPPT | SEQ ID NO:23 |
| 1.07% | VL-CDR3 | QQWLLGPPT | SEQ ID NO:24 |
| 1.04% | VL-CDR3 | QQWSTQRPT | SEQ ID NO:25 |
| 1.04% | VL-CDR3 | QQWSERPPT | SEQ ID NO:26 |
| 1.02% | VL-CDR3 | QQWSGQPPT | SEQ ID NO:27 |
| 1.02% | VL-CDR3 | QQWKCNPPT | SEQ ID NO:28 |
| 0.99% | VL-CDR3 | QQWRNAPPT | SEQ ID NO:29 |
| 0.99% | VL-CDR3 | QQWSIGLPT | SEQ ID NO:30 |
| 0.98% | VL-CDR3 | QQWKTSPPT | SEQ ID NO:31 |
| 0.97% | VL-CDR3 | QQWSYTSPT | SEQ ID NO:32 |
| 0.94% | VL-CDR3 | QQWHGKPPT | SEQ ID NO:33 |
| 0.93% | VL-CDR3 | QQWSTSEPT | SEQ ID NO:34 |

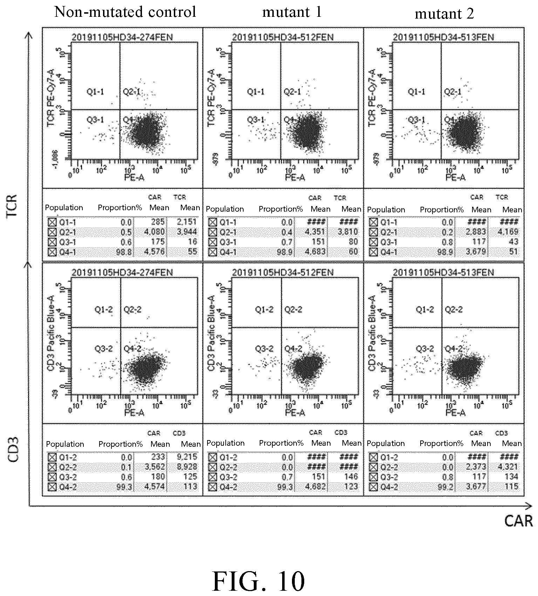

7) Mutant VL-CDR3: QQWSTQRPT (SEQ ID No: 25) and mutant VL-CDR3: QQWSGQPPT (SEQ ID No: 27) were selected for plasmid construction.
8) The relevant CAR-T indicators containing 2 mutants were detected following the procedure of above examples, and the results were shown in FIGS. 10-13.

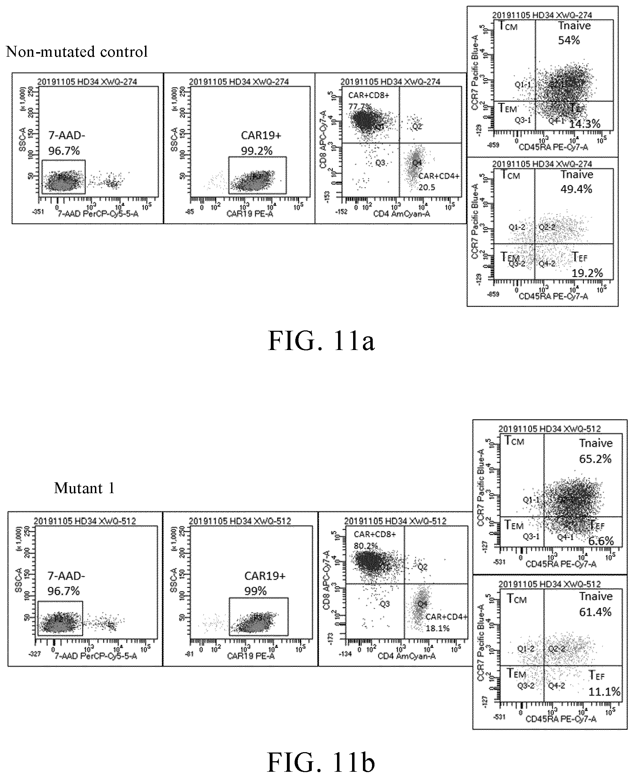

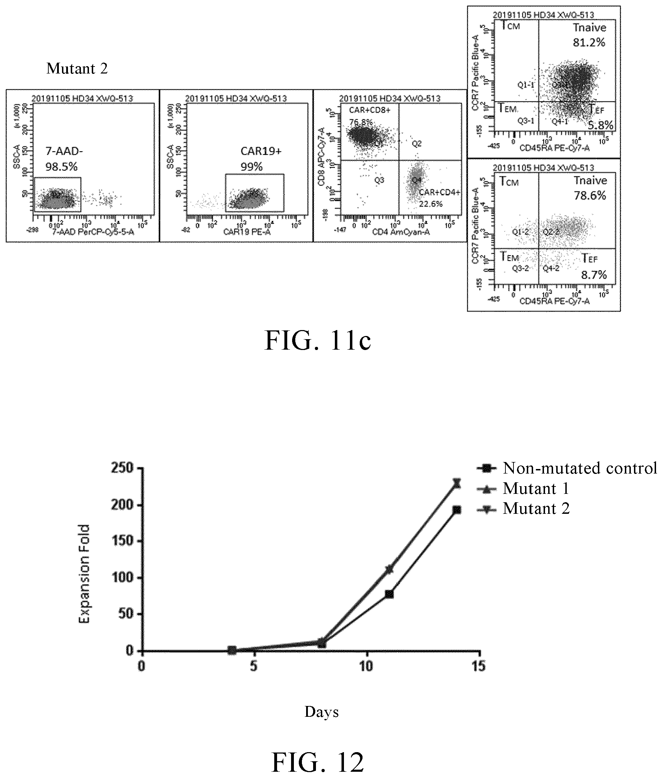

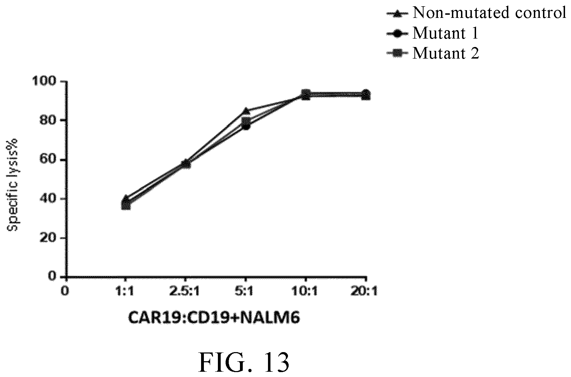

Referring to FIG. 10, mutants 1 and 2 exhibited the same degree of ability to inhibit the expression of TCR and CD3 on the cell surface as the non-mutated control. FIG. 11 showed that mutant 1 and mutant 2 exhibit the same proportions of T cell immunophenotypes as the non-mutated control. FIGS. 12-13 showed expansion capacity and tumor cell killing. As shown in FIG. 12, the mutants exhibited expansion efficiencies equivalent to the non-mutated control; as shown in FIG. 13, the mutants exhibited equivalent tumor cell killing ability to the non-mutated control.

These results showed that T cells containing mutant 1 and mutant 2 eliminated MHC-I epitopes present in the non-mutated antibody sequence compared with the non-mutated control, and the 2 mutants did not affect the immunophenotypic distribution, cell expansion, and chimeric antigen receptor-mediated killing of target cells compared with the control.

All documents mentioned in the present description are cited in the present application as references, as if each is cited separately as a reference. It is further understood that, after reading the above contents of the present description, those skilled in the art may make various changes or modifications to the present description, and these equivalent forms still fall within the scope defined by the claims appended to the present application.

**Sequence in the description**

| **name** | **SEQ ID NO.** | **name** | **SEQ ID NO.** |
|---|---|---|---|
| VH | SEQ ID NO:1 | LCDR3 mutant | SEQ ID NO:27 |
| VL | SEQ ID NO:2 | LCDR3 mutant | SEQ ID NO:28 |
| HCDR1 | SEQ ID NO:3 | LCDR3 mutant | SEQ ID NO:29 |
| HCDR2 | SEQ ID NO:4 | LCDR3 mutant | SEQ ID NO:30 |
| HCDR3 | SEQ ID NO:5 | LCDR3 mutant | SEQ ID NO:31 |
| LCDR1 | SEQ ID NO:6 | LCDR3 mutant | SEQ ID NO:32 |
| LCDR2 | SEQ ID NO:7 | LCDR3 mutant | SEQ ID NO:33 |
| LCDR3 | SEQ ID NO:8 | LCDR3 mutant | SEQ ID NO:34 |
| HCDR2 general | SEQ ID NO:9 | Retention sequence 1 | SEQ ID NO:35 |
| HCDR2 mutant | SEQ ID NO:10 | Retention sequence 2 | SEQ ID NO:36 |
| HCDR2 mutant | SEQ ID NO:11 | Retention sequence 3 | SEQ ID NO:37 |
| HCDR2 mutant | SEQ ID NO:12 | Retention sequence 4 | SEQ ID NO:38 |
| HCDR3 general | SEQ ID NO:13 | Retention sequence 5 | SEQ ID NO:39 |
| HCDR3 mutant | SEQ ID NO:14 | Retention sequence 6 | SEQ ID NO:40 |
| LCDR3 general | SEQ ID NO:15 | Linker | SEQ ID NO:41 |
| LCDR3 mutant | SEQ ID NO:16 | VH | SEQ ID NO:42 |
| LCDR3 mutant | SEQ ID NO:17 | VL | SEQ ID NO:43 |
| LCDR3 mutant | SEQ ID NO:18 | Amino acid sequence of exemplary expression frame | SEQ ID NO:44 |
| LCDR3 mutant | SEQ ID NO:19 | UCHT1-VL | SEQ ID NO:45 |
| LCDR3 mutant | SEQ ID NO:20 | UCHT1-VH | SEQ ID NO:46 |
| LCDR3 mutant | SEQ ID NO:21 | OKT3-VL | SEQ ID NO:47 |
| LCDR3 mutant | SEQ ID NO:22 | OKT3-VH | SEQ ID NO:48 |
| LCDR3 mutant | SEQ ID NO:23 | Primer 1 | SEQ ID NO:49 |
| LCDR3 mutant | SEQ ID NO:24 | Primer 2 | SEQ ID NO:50 |
| LCDR3 mutant | SEQ ID NO:25 | Exemplary nucleic acid sequence | SEQ ID NO:51 |
| LCDR3 mutant | SEQ ID NO:26 | | |

## Claims

1. An engineered T cell, wherein the T cell expresses a polypeptide that downregulates a cell surface TCR/CD3 complex, the polypeptide comprises a heavy chain variable region (VH) that has an amino acid sequence of SEQ ID No: 1 or more than 95% identical to SEQ ID No: 1, and a light chain variable region (VL) that has an amino acid sequence of SEQ ID No: 2 or more than 95% identical to SEQ ID No: 2.

2. The T cell according to claim 1, wherein
the VH of the polypeptide that downregulates the cell surface TCR/CD3 complex comprises a sequence of HCDR2: X₁X₂X₃X₄SGYT as shown in SEQ ID No: 9, wherein X₁, X₂, X₃, X₄ are any amino acid, or
the VH of the polypeptide that downregulates the cell surface TCR/CD3 complex comprises a sequence of HCDR3: ARSX₅X₆X₇DYDGFAY as shown in SEQ ID No: 13, wherein X₅, X₆, X₇ are any amino acid; or
the VL of the polypeptide that downregulates the cell surface TCR/CD3 complex comprises a sequence of LCDR3: QQWX₈X₉X₁₀X₁₁X₁₂T as shown in SEQ ID No: 15, wherein X₈, X₉, X₁₀, X₁₁, X₁₂ are any amino acid.

3. The T cell according to claim 2, wherein the polypeptide that downregulates the cell surface TCR/CD3 complex comprises has one or more features selected from the group consisting of:
in the HCDR2 as shown in sequence of SEQ ID No: 9, X₁ amino acid is serine, isoleucine, alanine, valine, leucine, isoleucine, proline, phenylalanine, methionine, tryptophan, threonine, glycine, asparagine, glutamine, tyrosine or cysteine, X₂ amino acid is threonine, asparagine, glycine, glutamine, serine, tyrosine, cysteine, valine or isoleucine, X₃ amino acid is proline, alanine, valine, leucine, isoleucine, phenylalanine, methionine, tryptophan, glycine, asparagine, glutamine, serine, threonine, tyrosine or cysteine, and X₄ amino acid is arginine, lysine, histidine, alanine, valine, leucine, isoleucine, proline, phenylalanine, methionine or tryptophan;
in the HCDR3 as shown in sequence of SEQ ID No: 13, X₅ amino acid is alanine, glycine, glutamine, serine, threonine, tyrosine, cysteine, lysine, arginine or histidine, X₆ amino acid is tyrosine, alanine, valine, leucine, isoleucine, phenylalanine, methionine, tryptophan, aspartic acid or glutamic acid, X₇ amino acid is tyrosine, lysine, histidine, glycine, asparagine, glutamine, serine, threonine, tyrosine, or cysteine;
in the LCDR3 as shown in sequence SEQ ID No: 15, X₈ amino acid is serine, alanine, glycine, asparagine, glutamine, threonine, tyrosine, cysteine, alanine, valine, leucine, isoleucine, proline, phenylalanine, methionine, tryptophan, lysine, arginine or histidine, X₉ amino acid is threonine, serine, glycine, asparagine, glutamine, tyrosine, cysteine, lysine, arginine, histidine, phenylalanine, tryptophan, alanine, valine, leucine, isoleucine, proline, methionine, aspartic acid or glutamic acid, X₁₀ amino acid is glutamine, asparagine, glycine, glutamine, serine, threonine, tyrosine, cysteine, valine, isoleucine, alanine, leucine, proline, phenylalanine, methionine, tryptophan, lysine, arginine or histidine, X₁₁ amino acid is proline, alanine, valine, leucine, isoleucine, phenylalanine, methionine, tryptophan, threonine, isoleucine, lysine, arginine, histidine, tyrosine, tryptophan, glycine, asparagine, glutamine, serine or cysteine, X₁₂ amino acid is glycine, asparagine, glutamine, serine, threonine, tyrosine, cysteine, alanine, valine, leucine, isoleucine, proline, phenylalanine, methionine, tryptophan.

4. The T cell according to any of claims 1-3, wherein the polypeptide that downregulates the cell surface TCR/CD3 complex comprises one or more of each of the following groups:
(1) One of the following LCDR3 sequences: SEQ ID No: 16, SEQ ID No: 17, SEQ ID No: 18, SEQ ID No: 19, SEQ ID No: 20, SEQ ID No: 21, SEQ ID No: 22, SEQ ID No: 23, SEQ ID No: 24, SEQ ID NO: 25, SEQ ID No: 26, SEQ ID No: 27, SEQ ID No: 28, SEQ ID No: 29, SEQ ID No: 30, SEQ ID No: 31, SEQ ID No: 32, SEQ ID No: 33, SEQ ID No: 34,
(2) One of the following HCDR2 sequences: SEQ ID NO: 10, SEQ ID NO: 11, SEQ ID NO: 12,
(3) HCDR3 sequence: SEQ ID NO: 14.

5. The T cell according to any of claims 1-4, wherein the polypeptide is a single chain antibody.

6. The T cell according to any of claims 1-5, wherein the polypeptide that downregulates the cell surface TCR/CD3 complex comprises a localization sequence and optionally a signal peptide, preferably,
the localization sequence is selected from the group consisting of endoplasmic reticulum retention sequence, Golgi retention sequence, E3 ubiquitin ligase binding sequence, proteasome localization sequence, or lysosome localization sequence; preferably, the endoplasmic reticulum retention sequence comprises or consists of any of the sequences shown in SEQ ID Nos: 35-40, where X is any amino acid,
a linker is included between the localization sequence and the antibody, preferably as shown in SEQ ID No: 41;
the signal peptide is shown in positions 516-537 of SEQ ID No: 44.

7. The T cell according to any of claims 1-6, wherein the T cell further expresses a therapeutic protein, preferably the therapeutic protein is a chimeric antigen receptor.

8. The T cell according to claim 7, wherein the T cell contains an expression frame of the therapeutic protein and an expression frame of the polypeptide that downregulates the cell surface TCR/CD3 complex, or a coding sequence of the therapeutic protein and a coding sequence of the polypeptide that downregulates the cell surface TCR/CD3 complex are in the same expression frame.

9. An engineered T cell, wherein said T cell expresses a polypeptide that downregulates a cell surface TCR/CD3 complex, the polypeptide comprises: an antibody or an antigen binding fragment thereof, or a mutant having at least 90% sequence identity with the antibody or the antigen binding fragment thereof and retaining antigen binding activity of the antibody or the antigen binding fragment thereof; wherein, the antibody comprises a heavy chain variable region and a light chain variable region,
the heavy chain variable region has the following HCDRs:
HCDR1 as shown in SEQ ID NO:3: GYTFISYT,
HCDR2 as shown in SEQ ID NO:9: X₁X₂X₃X₄SGYT, wherein X₁, X₂, X₃, X₄ are any amino acid,
HCDR3 as shown in SEQ ID NO:13: ARSX₅X₆X₇DYDGFAY, wherein X₅, X₆, X₇ are any amino acid,
the light chain variable region has the following LCDRs:
LCDR1 as shown in SEQ ID NO:6: SSVSY,
LCDR2 as shown in SEQ ID NO:7: DTS,
LCDR3 as shown in SEQ ID NO:15: QQWX₈X₉X₁₀X₁₁X₁₂T, wherein X₈, X₉, X₁₀, X₁₁, X₁₂ are any amino acid.

10. The T cell according to claim 9, wherein the polypeptide that downregulates the cell surface TCR/CD3 complex has one or more features selected from the group consisting of:
X₁ is serine, isoleucine, alanine, valine, leucine, isoleucine, proline, phenylalanine, methionine, tryptophan, threonine, glycine, asparagine, glutamine, tyrosine, or cysteine,
X₂ is threonine, asparagine, glycine, glutamine, serine, tyrosine, cysteine, valine, or isoleucine,
X₃ is proline, alanine, valine, leucine, isoleucine, phenylalanine, methionine, tryptophan, glycine, asparagine, glutamine, serine, threonine, tyrosine, or cysteine,
X₄ is arginine, lysine, histidine, alanine, valine, leucine, isoleucine, proline, phenylalanine, methionine, or tryptophan,
X₅ is alanine, glycine, glutamine, serine, threonine, tyrosine, cysteine, lysine, arginine, or histidine,
X₆ is tyrosine, alanine, valine, leucine, isoleucine, phenylalanine, methionine, tryptophan, aspartate, or glutamate,
X₇ is tyrosine, lysine, histidine, glycine, asparagine, glutamine, serine, threonine, tyrosine, or cysteine,
X₈ is serine, alanine, glycine, asparagine, glutamine, threonine, tyrosine, cysteine, alanine, valine, leucine, isoleucine, proline, phenylalanine, methionine, tryptophan, lysine, arginine, or histidine,
X₉ is threonine, serine, glycine, asparagine, glutamine, tyrosine, cysteine, lysine, arginine, histidine, phenylalanine, tryptophan, alanine, valine, leucine, isoleucine, proline, methionine, aspartic acid, or glutamic acid,
X₁₀ is glutamine, asparagine, glycine, glutamine, serine, threonine, tyrosine, cysteine, valine, isoleucine, alanine, leucine, proline, phenylalanine, methionine, tryptophan, lysine, arginine, or histidine,
X₁₁ is proline, alanine, valine, leucine, isoleucine, phenylalanine, methionine, tryptophan, threonine, isoleucine, lysine, arginine, histidine, tyrosine, tryptophan, glycine, asparagine, glutamine, serine, or cysteine,
X₁₂ is glycine, asparagine, glutamine, serine, threonine, tyrosine, cysteine, alanine, valine, leucine, isoleucine, proline, phenylalanine, methionine, tryptophan.

11. The T cell according to claims 9 or 10, wherein the polypeptide that downregulates the cell surface TCR/CD3 complex has one or more features selected from the group consisting of:
LCDR3 is selected from the group consisting of: SEQ ID NO:16, SEQ ID NO:17, SEQ ID NO: 18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34,
HCDR2 is selected from the group consisting of: SEQ ID NO: 10, SEQ ID NO:11, SEQ ID NO: 12,
HCDR3 is SEQ ID NO:14.

12. The T cell according to any of claims 9-11, wherein,
the amino acid sequence of the heavy chain variable region is selected from the group consisting of:
(1) SEQ ID NO:42,
(2) a variant of SEQ ID No: 42 containing HCDR2 and/or HCDR3 as described in claims 10 or 11,
(3) sequences with more than 95% identity to (1) or (2), and/or
the amino acid sequence of the light chain variable region is selected from the group consisting of:
(1) SEQ ID NO:43,
(2) a variant of SEQ ID No: 43 containing a sequence of LCDR3 as described in claims 10 or 11,
(3) a sequence with more than 95% identity to (1) or (2).

13. The T cell according to any of claims 9-12, wherein the antibody is a single chain antibody.

14. The T cell according to any of claims 9-12, wherein the polypeptide that downregulates the cell surface TCR/CD3 complex comprises a localization sequence and optionally a signal peptide, preferably,
the localization sequence is selected from the group consisting of: endoplasmic reticulum retention sequence, Golgi retention sequence, E3 ubiquitin ligase binding sequence, proteasome localization sequence, or lysosome localization sequence; preferably, the endoplasmic reticulum retention sequence comprises or consists of any of the sequences shown in SEQ ID Nos: 35-40, where X is any amino acid;
a linker is included between the localization sequence and the antibody, preferably as shown by SEQ ID No: 41,
the signal peptide is shown in positions 516-537 of SEQ ID No: 44.

15. The T cell according to any of claims 9-14, further expresses a therapeutic protein; preferably, the therapeutic protein is a chimeric antigen receptor.

16. The T cell according to claim 15, wherein the T cell contains an expression frame of the therapeutic protein and an expression frame of the polypeptide that downregulates the cell surface TCR/CD3 complex, or a coding sequence of the therapeutic protein and a coding sequence of the polypeptide that downregulates the cell surface TCR/CD3 complex are in the same expression frame.

17. An antibody or an antigen binding fragment thereof, wherein the antibody has an amino acid sequence of HCDR1 of GYTFISYT, an amino acid sequence of HCDR2 of X₁X₂X₃X₄SGYT, an amino acid sequence of HCDR3 of ARSX₅X₆X₇DYDGFAY, an amino acid sequence of LCDR1 of SSVSY, an amino acid sequence of LCDR2 of DTS, an amino acid sequence of LCDR3 of QQWX₈X₉X₁₀X₁₁X₁₂T; wherein, X₁-X₁₂ are as described in claim 10, and HCDR2 is not INPRSGYT, HCDR3 is not ARSAYYDYDGFAY, LCDR3 is not QQWSSNPPT,
preferably, the LCDR3, HCDR2 and HCDR3 of the antibody are as described in claim 11.

18. The antibody or the antigen binding fragment thereof according to claim 17, wherein
the amino acid sequence of the heavy chain variable region of the antibody is shown in SEQ ID No: 42, but the sequences of its HCDR2 and/or HCDR3 are as described in claims 10 or 11;
the amino acid sequence of the light chain variable region of the antibody is shown in SEQ ID No: 43, but the sequence of its LCDR3 is as described in claims 10 or 11.

19. A fusion protein comprising a localization sequence, a polypeptide that downregulates a cell surface TCR/CD3 complex, and, optionally, a signal peptide; preferably, the polypeptide that downregulates the cell surface TCR/CD3 complex is as described in any of claims 9-18, preferably,
the signal peptide is shown in positions 516-537 of SEQ ID No: 44,
a linker is included between the localization sequence and the polypeptide that downregulates the cell surface TCR/CD3 complex, preferably as shown by SEQ ID No: 41,
the fusion proteins are as shown in positions 516-804 of SEQ ID No: 44 or as shown in positions 538-804 of SEQ ID No: 44.

20. A nucleic acid molecule, wherein the nucleic acid molecule is selected from the group consisting of:
(1) a nucleic acid molecule comprising a coding sequence of a therapeutic protein and a coding sequence of a polypeptide that downregulates a cell surface TCR/CD3 complex;
(2) the coding sequence of the fusion protein according to claim 19;
(3) the coding sequence of the antibody or the antigen binding fragment thereof of claims 17 or 18; and
(4) a complementary sequence or fragment of (1), (2), or (3);
preferably, the therapeutic protein is a chimeric antigen receptor, and the polypeptide that downregulates the cell surface TCR/CD3 complex is according to claims 9-14.

21. A nucleic acid construct, wherein the nucleic acid construct comprises a nucleic acid molecule according to claim 20,
preferably, the nucleic acid construct contains an expression frame of the therapeutic protein and an expression frame of the polypeptide that downregulates the cell surface TCR/CD3 complex; or the nucleic acid construct is one expression frame wherein a coding sequence of the therapeutic protein and a coding sequence of the polypeptide that downregulates the cell surface TCR/CD3 complex are in this expression frame; or
the nucleic acid construct is a cloning vector or expression vector.

22. A lentivirus wherein the lentivirus contains a nucleic acid molecule according to claim 20 or a nucleic acid construct according to claim 21.

23. A host cell, wherein the host cell comprises, expresses and/or secrets an antibody or an antigen binding fragment thereof according to claims 17 or 18, or a fusion protein according to claim 19, and optionally a therapeutic protein; preferably, the host cell comprises a nucleic acid molecule according to claim 20, a nucleic acid construct according to claim 21 and/or a lentivirus according to claim 22.

24. A pharmaceutical composition comprising a T cell according to any of claims 9-16, an antibody or an antigen binding fragment thereof according to claims 17 or 18, a fusion protein according to claim 19, a nucleic acid molecule according to claim 20, a nucleic acid construct according to claim 21 and/or a lentiviruse according to claim 22, and a pharmaceutically acceptable carrier.

25. Use of a polypeptide that downregulates a cell surface TCR/CD3 complex or its coding sequence, a nucleic acid molecule according to claim 20, a nucleic acid construct according to claim 21 and/or a lentivirus according to claim 22 in the preparation of a T cell with downregulated cell surface TCR, or in the preparation of T cells for cancer therapy, wherein the polypeptide that downregulates a cell surface TCR/CD3 complex is as described in claims 9-14.
